# EUROPEAN PATENT SPECIFICATION

(11) **EP 1 988 971 B1**
(45) Date of publication and mention of the grant of the patent: **28.02.2018**
(21) Application number: 06845640.9
(22) Date of filing: 15.12.2006
(51) Int. Cl.: A61K 31/19, A61K 31/205, A61K 31/215, A61K 31/22, A23L 33/115, A61P 25/28

(54) **COMPOSITIONS AND METHODS FOR PRESERVING BRAIN FUNCTION**
ZUSAMMENSETZUNGEN UND VERFAHREN ZUR ERHALTUNG DER GEHIRNFUNKTION
COMPOSITIONS ET PROCEDES DE PRESERVATION DES FONCTIONS CEREBRALES

(30) Priority: 15.12.2005 US 751391 P
(43) Date of publication of application: 12.11.2008
(73) Proprietor: Nestec S.A., 1800 Vevey (CH)
(72) Inventor: LARSON, Brian, T., Dowling, Michigan 49050 (US); HENDERSON, Samuel, T., Broomfield, CO 80020 (US); ROBERTS, Matthew, A., 1616 Attalens (CH)
(74) Representative: Rupp, Christian
(86) International application number: PCT/US2006/048077
(87) International publication number: WO 2007/070701

(56) References cited:
- EP-A- 1 350 435
- WO-A-2005/006890
- WO-A-2007/001883
- WO-A-2007/115282
- WO-A2-2005/025322
- WO-A2-2005/027822
- GB-A- 2 084 172
- US-A1- 2005 070 602
- US-B1- 6 835 750
- DATABASE WPI Week 199445 Thomson Scientific, London, GB; AN 1994-363533 XP002497566 & JP 06 287138 A (GREEN CROSS CORP) 11 October 1994 (1994-10-11)
- DATABASE CA [Online] CHEMICAL ABSTRACTS SERVICE, COLUMBUS, OHIO, US SUZUKI, SHIGEO: 'Digestion of the foods. V. The digestion of synthetic fats. 3' Retrieved from STN Database accession no. 1935:25945
- VERKADE P E ET AL: "Fat metabolism: X. Feeding dogs with single acid-saturated triglycerides", PROCEEDINGS OF THE SECTION OF SCIENCES / KONINKLIJKE NEDERLANDSCHE AKADEMIE VAN WETENSCHAPPEN, AMSTERDAM : NOORD-HOLLANDSCHE UITG. MIJ, NL, 1 January 1937 (1937-01-01), pages 411-413, XP009186114, ISSN: 0370-0348
- S H Chiang ET AL: "LIMITS OF MEDIUM-CHAIN AND LONG-CHAIN TRIACYLGLYCEROL UTILIZATION BY NEONATAL PIGLETS112", JOURNAL OF ANIMAL SCIENCE, vol. 68, no. 6, 1 June 1990 (1990-06-01), XP055214484, US ISSN: 0021-8812, DOI: 10.2527/1990.6861632x
- NIELSEN N S ET AL: "Effect of structured lipids based on fish oil on the growth and fatty acid composition in rainbow trout (Oncorhynchus mykiss)", AQUACULTURE, ELSEVIER, AMSTERDAM, NL, vol. 250, no. 1-2, 14 November 2005 (2005-11-14), pages 411-423, XP027614770, ISSN: 0044-8486 [retrieved on 2005-11-14]
- [Online] Retrieved from the Internet: <URL:http://www.stepan.com/uploadedFiles/Li terature_and_Downloads/General_Lit/Food,_Nu trition_and_Pharmaceutical/NEOBEEBrochure.p df> [retrieved on 2015-10-05]
- REGER M A ET AL: "Effect of .beta.-hydroxybutyrate on cognition in memory-impaired adults", NEUROBIOLOGY OF AGING, TARRYTOWN, NY, US, vol. 25, no. 3, 1 March 2004 (2004-03-01), pages 311-314, XP008122713, ISSN: 0197-4580, DOI: 10.1016/S0197-4580(03)00087-3
- HENDERSON SAMUEL T ET AL: "Study of the ketogenic agent AC-1202 in mild to moderate Alzheimer's disease: a randomized, double-blind, placebo-controlled, multicenter trial", NUTRITION & METABOLISM, BIOMED CENTRAL. LONDON, GB, vol. 6, no. 1, 10 August 2009 (2009-08-10) , page 31, XP021059726, ISSN: 1743-7075, DOI: 10.1186/1743-7075-6-31

## Description

### FIELD OF THE INVENTION

The present Invention is related to mammalian nutrition and effects thereof on cognitive function, behavior, and brain physiology. In particular, the present invention utilizes medium chain triglycerides, administered as part of a long-term dietary regimen, to preserve or improve learning, attention, motor performance, cerebrovascular function, social behavior, and to increase activity levels, particularly in aging animals.

### BACKGROUND OF THE INVENTION

Various publications, including patents, published applications, technical articles and scholarly article are cited throughout the specification. Each of these cited publications is incorporated by preference herein, in its entirety.

Cognitive impairment, progressive decline in cognitive function, changes in brain morphology, and changes in cerebrovascular function are commonly observed in aged or aging individuals. Age-related or age-associated cognitive impairment may manifest itself in many ways, and can include short-term memory loss, diminished capacity to learn or rate of learning, diminished attention, diminished motor performance, and/or dementia, among other indicia. In some cases, a specific etiology of such cognitive decline is unknown, while on other cases, cognitive impairment stems from the onset or progression of recognized diseases, disorders, or syndromes, for example, Alzheimer's Disease (AD). Age-associated cognitive decline is distinct from, and can occur independently of AD.

The primary energy source of the healthy mammalian brain is glucose. Age-related cognitive decline has been correlated with impaired glucose metabolism. (Finch CE *et al.,* 1997). Impaired glucose metabolism can produce an energy deficit in the brain, and may result in neuronal loss and morphological changes in the brain. (Hoyer S., 1990).

Impaired glucose metabolism can diminish the ability of cells to repair and resist oxidative damage. (Munch G *et al.,* 1998). The concomitant neuronal loss and morphological abnormalities appear to contribute to the reduced mental capacity in the aged.

Alzheimer's patients also exhibit decreased glucose metabolism, and positron emission tomography studies have shown decreased levels of cerebral glucose. (Drzezga A *et al.,* 2005; and, Small GW *et al.,* 2000). Although the precise mechanisms underlying the decrease in glucose levels and glucose metabolism is not fully understood, neuropathological events such as oxidative stress, neuronal cell death, and decreased levels of acetylcholine, ATP, and cholesterol, have all been correlated with decreased energy and glucose metabolism in the brain. (Swaab *et al.,* 1998).

In addition to the effects of changes in glucose metabolism, according to one hypothesis, a reduction in the regional blood flow to the brain contributes to cognitive decline and dementia in humans (Wardlaw JM *et al.,* 2003). Regional cerebral blood volume is affected by human age and stage of dementia (Split A *et al.,* 2005; and, Petrella JR *et al.,* 1998).

Although glucose is understood to be the primary energy source of the mammalian brain, it has long been known that in periods of prolonged fasting or carbohydrate deficiency, ketones bodies can serve as an alternative energy source in the brain. Ketone bodies, including acetone, acetoacetate, β-hydroxybutyrate, can be readily used by mitochondria for ATP generation, and may exert a protective effect on neurons from free radical damage. (VanItallie TB *et al.,* 2003).

Ketone bodies have been proposed for use in AD patients. (Reger MA *et al.,* 2004; VanItallie TB *et al.,* 2003; and U.S. Patent Nos. 6,323,237 and 6,316,038). Ketone bodies have been used to treat dementia and Alzheimer's disease. For example, United States Patent Nos. 6,323,237 and 6,316,038 describe the use of ketone bodies and metabolic precursors of ketone bodies to treat neurodegenerative disorders.

Medium chain triglycerides (MCTs), are composed of fatty acid chains esterified to a glycerol backbone. MCTs, under some physiological circumstances, are metabolized to ketone bodies in the liver, however the MCTs must undergo metabolic processing before conversion to ketone bodies. After ingestion, the esterified fatty acids are cleaved from the MCT by lipases such as pancreatic and gastrointestinal lipases, the released medium chain fatty acids transported as free fatty acids via the portal vein to the liver. The medium chain fatty acids are not incorporated into chylomicrons as longer chain fatty acids are. In the liver, the medium chain fatty acids are oxidized to form acetyl-CoA. Accordingly, ketone bodies produced from MCTs can provide an alternative energy source to supplement the energy deficit in neuronal cells of Alzheimer's patients (Reger MA *et al.,* 2004). But unlike the ketone esters described in the foregoing US patents (United States Patent Nos. 6,323,237 and 6,316,038), which are metabolic equivalents of ketone bodies (e.g. polymers of β hydroxybutyrate, and the like) that can be directly converted into ketone bodies, MCTs cannot be considered metabolically equivalent to ketone bodies because ingestion of MCTs does not always lead to the production of ketone bodies. In addition, where MCTs are converted into ketone bodies, it is through the condensation of two acetyl-CoA molecules, each of which may be derived from a variety of sources.

Animal models of cognitive impairment greatly facilitate the study of such conditions including their physiology, neurology, anatomy, and pathology. Dogs provide a useful model as they demonstrate a pattern of age-associated cognitive decline in learning and memory, variable as to function of cognitive task (Adams B *et al.,* 2000a; Chan ADF *et al.,* 2002; Su M-Y *et al.,* 1998; and, Tapp PD *et al.,* 2003). While the study of such decline in dogs as companion animals is useful in its own right, the fact that the observed decline mirrors age-related cognitive declines seen in humans (Adams B *et al.* 2000b) makes the studies even more valuable. Dogs also experience age-related reduction in regional cerebral metabolic rates for glucose (London ED *et al.,* 1983). Dogs exhibit age-dependent changes in regional cerebral blood volume and blood-brain barrier permeability that may be related to changes in cognition, brain structure, and neuropathology with age (Tapp PD *et al.,* 2005; and, Su MY, 1998). Aged dogs develop neuropathology that is related to that seen in both successfully aging humans and patients with AD, such as beta amyloid protein (Cotman CW and Berchtold, 2002; and Cummings BJ *et al.,* 1996). However, dogs do not demonstrate every hallmark of AD, in particular, tau-containing neurofibrillar tangles (Dimakopoulos AC *et al.,* 2002) have not been observed. Therefore, the condition in dogs is distinct and referred to as Canine Cognitive Dysfunction Syndrome (CCDS).

Both healthy aging or geriatric dogs, as well as those diagnosed with CCDS, may present clinically with progressive cognitive impairment and neuropathological changes (London ED *et al.,* 1983). In addition, both aging/geriatric dogs and those diagnosed with CCDS exhibit various behavioral disorders. For example, they may not respond to their name or familiar commands, may get lost or confused even in familiar surroundings, may no longer greet or respond to their owners or visitors, may exhibit diminished daytime activity, may walk in circles, may shun affection, and may lose bladder or bowel control.

There is thus a need in the art to develop compositions and methods for the treatment and/or prevention of cognitive impairment, particularly in aging or geriatric animals and in animals suffering from CCDS-like symptoms. In the case of companion animals, such therapies would be useful to improve the overall quality of life, to improve owner satisfaction, and to improve the bonds between the owner and companion animal.

US 6,835,750 B1 describes methods and compositions for treating or preventing the occurrence of senile dementia of the Alzheimer type or other conditions arising from reduced neuronal metabolism and leading to lessened cognitive function.

### SUMMARY OF THE INVENTION

The present invention relates to a composition comprising medium chain triglycerides (MCTs) composed of fatty acids, each independently having 5-12 carbons, esterified to a glycerol backbone for use on an extended regular basis in preventing, reducing, or delaying decline in one or more of cognitive function, motor performance and cerebrovascular function in an aging mammal that has exceeded 50% of the average life span of this particular species, wherein said composition is used at least once dally for periods in excess of two months and wherein the mammal is a dog or a cat as well as the use of such a composition.

The MCTs typically
are composed of fatty acids, each independently having 5-12 carbons, esterified to the glycerol backbone.

In some instances, greater than about 95% of the fatty acids are 8 carbons in length. The remaining fatty acids can be 6-carbon or 10-carbon fatty acids. In certain embodiments, the composition comprises at least about 1% to about 30% MCTs on a dry weight basis. The aforementioned composition can be a food composition, further comprising on a dry weight basis about 15-50% protein, 5-40% fat, 5-10% ash content, and having a moisture content of 5-20%.

The compositions are formulated for consumption by a dog or cat.

The composition may be formulated for administration to a healthy aging mammal. In certain embodiments, the mammal has a phenotype associated with age-related cognitive impairment. Such a phenotype can include one or more of decreased ability to recall, short-term memory loss, decreased learning rate, decreased capacity for learning, decreased problem solving skills, decreased attention span, decreased motor performance, increased confusion, or dementia, as compared to a control mammal not having the phenotype.

Further described is a method for preventing, reducing, or delaying decline in at least one of cognitive function, motor function, cerebrovascular function, or behavior in an aging mammal comprising the steps of: (1) identifying an aging mammal having, or at risk of, decline in at least one of cognitive function, motor function, cerebrovascular function, or behavior; and (2) administering to the mammal on an extended regular basis a composition comprising medium chain triglycerides (MCTs), as described above, in an amount effective to prevent, reduce, or delay decline in at least one of cognitive function, motor function, cerebrovascular function, or behavior in the mammal wherein the composition increases the circulating concentration of at least one ketone body in the mammal. In certain instances, the method further comprises the step of monitoring the ketone body concentrations in the mammal. In certain embodiments, the amount of each of β-hydroxybutyrate, acetoacetate and acetone is raised in the blood of the mammal.

In another embodiment, the composition comprises MCTs in an amount effective for lowering the amount in the blood of the mammal of one or more of alanine, branched chain amino acids, total lipoproteins, unsaturated fatty acids, or VLDL. In a particular embodiment, each of alanine, branched chain amino acids, total lipoproteins, unsaturated fatty acids, and VLDL is lowered in blood of the mammal.

In yet another embodiment, the composition comprises MCTs in an amount effective for raising an amount in the blood of the mammal of one or more of glutamine, phenylalanine, HDL, or citrate. In a particular embodiment, the amount of each of glutamine, phenylalanine, HDL, and citrate is raised in the blood of the animal.

In another embodiment, the composition comprises MCTs in an amount effective for improving blood flow to the brain. Additionally or alternatively, the composition comprises MCTs in an amount effective for improving the integrity of the blood brain barrier.

In another embodiments, the composition comprises MCTs in an amount effective for lowering blood urea nitrogen or decreasing protein degradation. In another embodiments, the composition comprises MCTs in an amount effective for lowering the amount or activity of alanine aminotransferase.

In accordance with this aspect of the invention, the composition used to the mammal can be a pet food or dietary supplement. The mammal is a dog or a cat.

It is described that the above-described method calls for administration of a composition comprising between about 1% and about 30% MCTs on a dry weight basis. The composition is used on a regular basis, which, in one embodiment, is at least once daily. The composition is used as part of a daily dietary regimen for at least about two month or at least about three months or longer, up to the duration of the mammal's life.

Further described is a method for preventing, reducing, or delaying decline in at least one of cognitive function, motor function, cerebrovascular function, or behavior in an aging mammal comprising the steps of: (1) identifying an aging mammal not having an age-related cognitive impairment disease; and (2) administering to the mammal, on an extended regular basis, a composition comprising medium chain triglycerides (MCTs), as described above, in an amount effective to prevent, reduce, or delay decline in at least one of cognitive function, motor function, cerebrovascular function, or behavior in the mammal, (3) measuring the concentration of at least one ketone body, and at least one of cognitive function, motor function, cerebrovascular function, or behavior in the mammal at least periodically for the duration of the administering step; (4) compacting the at least one ketone body concentration and the measure of cognitive function, motor function, cerebrovascular function, or behavior to that of a control animal not receiving the administered composition; and (5) correlating the ketone body concentration with the measure of cognitive function, motor function, cerebrovascular function, or behavior thereby establishing the prevention, reduction, or delay of the decline of at least one of cognitive function, motor function, cerebrovascular function, or behavior as a result of the administration of the composition.

T In certain embodiments, the amount of each of β-hydroxybutyrate, acetoacetate and acetone is raised in the blood of the mammal.

In another embodiment, the composition comprises MCTs in an amount effective for lowering the amount in the blood of the mammal of one or more of alanine, branched chain amino acids, total lipoproteins, unsaturated fatty acids, or VLDL. In a particular embodiment, each of alanine, branched chain amino acids, total lipoproteins, unsaturated fatty acids, and VLDL is lowered in blood of the mammal.

In yet another embodiment, the composition comprises MCTs in an amount effective for raising an amount in the blood of the mammal of one or more of glutamine, phenylalanine, HDL, or citrate. In a particular embodiments, the amount of each of glutamine, phenylalanine, HDL, and citrate is raised in the blood of the animal.

In another embodiment, the composition comprises MCTs in an amount effective for improving blood flow to the brain. Additionally or alternatively, the composition comprises MCTs in an amount effective for improving the integrity of the blood brain barrier.

In another embodiment, the composition comprises MCTs in an amount effective for lowering blood urea nitrogen or decreasing protein degradation. In another embodiments, the composition comprises MCTs in an amount effective for lowering the amount or activity of alanine aminotransferase.

The compositions used to the mammal can be a pet food, or dietary supplement. The mammal is a dog or cat.

It is described that the composition comprises MCTs in an amount effective for improving social behaviors of the companion animal.

It is described that the above-described method calls for administration of a compositions comprising between about 1% and about 30% MCTs on a dry weight basis. The composition is administered on a regular basis, which, in one embodiment, is at least once daily. The composition is administered as part of a daily dietary regimen for at least two months or at least about three months or longer, up to the duration of the mammal's life.

Further described is a method for preventing, reducing, or delaying decline in at least one of cognitive function, motor function, cerebrovascular function, or behavior in a population or healthy aging mammals comprising the steps of: (1) identifying a population of healthy aging mammals not having age-related cognitive impairment; (2) dividing the population into at least a control group and one or more test groups (3) formulating at least one diet-based delivery system for delivering a composition comprising medium chain triglycerides (MCTs), as described above, in an amount effective for elevating and maintaining an elevated level of Ketone bodies in the blood of an individual mammal, wherein, on an extended regular basis, each test group receives a formulation delivering a composition comprising MCTs and the control group does not receive any composition comprising MCTs; (4) comparing at least one of cognitive function motor function, cerebrovascular function, or behavior in the control and test groups; (5) determining which of the diet-based delivery systems for delivering the composition comprising MCTs was effective in preventing, reducing, delaying decline of at least one of cognitive function motor function, cerebrovascular function, or behavior, and (6) administering the diet-based delivery system determined in step (e) to a population of aging mammals, thereby preventing, reducing, delaying decline in at least one of cognitive function, motor function, cerebrovascular function, or behavior. As described in greater detail herein, the extended regular basis can extend from at least one week up to a year or longer. It is described that the amount of each of β-hydroxybutyrate, acetoacetate and acetone is raised in the blood of the mammal.

In another embodiment, the composition comprises MCTs in an amount effective for lowering the amount in the blood of the mammal of one or more of alanine, branched chain amino acids, total lipoproteins, unsaturated fatty acids, or VLDL. In a particular embodiment, each of alanine, branched chain amino acids, total lipoproteins, unsaturated fatty acids, and VLDL is lowered in blood of the mammal.

In yet another embodiment, the composition comprises MCTs in an amount effective for raising an amount in the blood of the mammal of one or more of glutamine, phenylalanine, HDL, or citrate. In a particular embodiments, the amount of each of glutamine, phenylalanine, HDL, and citrate is raised in the blood of the animal.

In another embodiments, the composition comprises MCTs in an amount effective for improving blood flow to the brain. Additionally or alternatively, the composition comprises MCTs in an amount effective for improving the integrity of the blood brain barrier.

In another embodiment, the composition comprises MCTs in an amount effective for lowering blood urea nitrogen or decreasing protein degradation. In another embodiments, the composition comprises MCTs in an amount effective for lowering the amount or activity of alanine aminotransferase.

In accordance with this aspect of the invention, the composition used to the mammal can be a pet food, or dietary supplement. The mammal is a dog or cat.

It is described that the above-described method calls for administration of a composition comprising between about 1% and about 30% MCTs on a dry weight basis. The composition is used on a regular basis, which, is at least once daily. the composition is ; used as part of a daily dietary regimen for at least; two months or at least about three months up to at least about a year longer, extending to the duration of the mammal's life.

Other features and advantages of the invention will become apparent by reference to the drawings, detailed description and examples that follow.

### BRIEF DESCRIPTION OF THE DRAWINGS

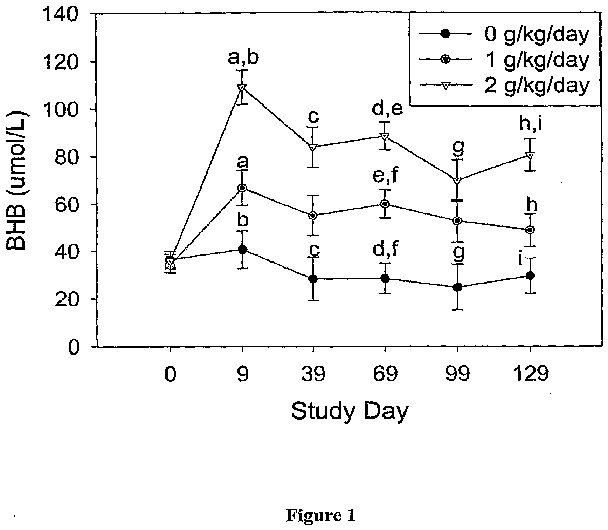

**Figure 1****. Average Circulating BHB Concentration Over Time.** A graph of blood BHB concentrations (µmol/liter) at time points during the study. Symbols represent the following treatment groups: dark circles = control group (0 g MCT/kg body weight/day); light circles = 1 g MCT/kg body weight/day; triangles = 2 g MCT/kg body weight/day. Identical letters indicate statistically significant differences.

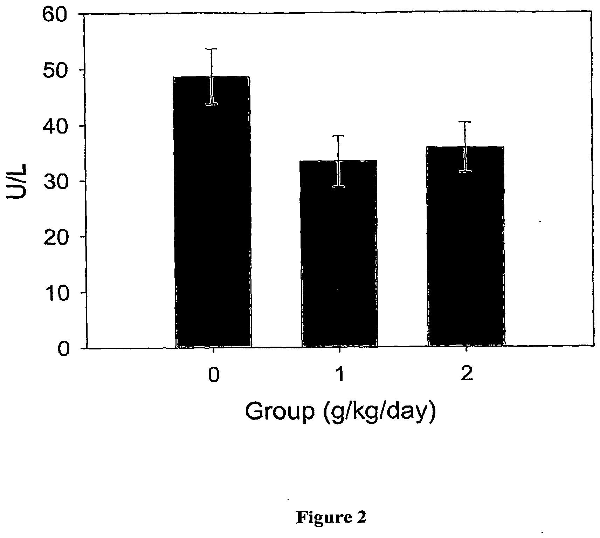

**Figure 2****. Alanine Aminotransferase Enzyme Activity As a Function of MCT Provided in the Diet.** A graph of the activity (U/L) of the enzyme, alanine aminotransferase ("ALT"), shows that activity varied with the MCT dose provided. Each bar represents a study group receiving a specific dosage of MCTs provided with the dietary regimen (0, 1, or 2 g MCT/kg body weight/day). ALT activity tended to be highest in the control group not receiving MCTs (0 g MCT/kg body weight/day).

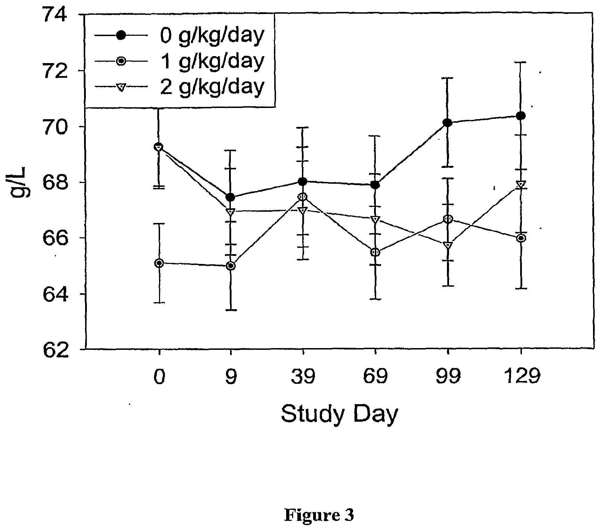

**Figure 3****. Total Protein Concentration in the Blood Over the Course of the Study.** The figure shows changes in amount of total protein (g/L) in the samples over time for each of the dietary groups. Symbols represent the following treatment groups: dark circles = control group (0 g MCT/kg body weight/day); light circles = 1 g MCT/kg body weight/day; triangles = 2 g MCT/kg body weight/day. Total protein was lower in the I and 2 g/kg/day groups, compared to the 0 g/kg/day. These differences were especially apparent at the end of the study.

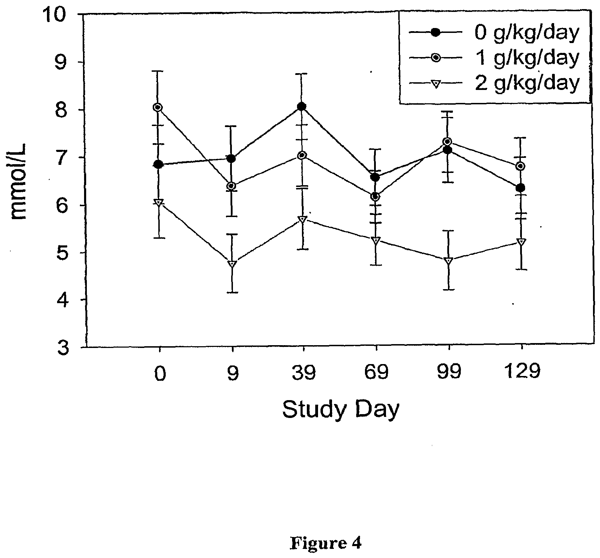

**Figure 4****. Blood Urea Nitrogen Concentrations Over Time.** Blood Urea Nitrogen (BUN) concentrations tended to be lowest in the 2 g/kg/day treatment group. Symbols represent the following treatment groups: dark circles = control group (0 g MCT/kg body weight/day); light circles = 1 g MCT/kg body weight/day; triangles = 2 g MCT/kg body weight/day.

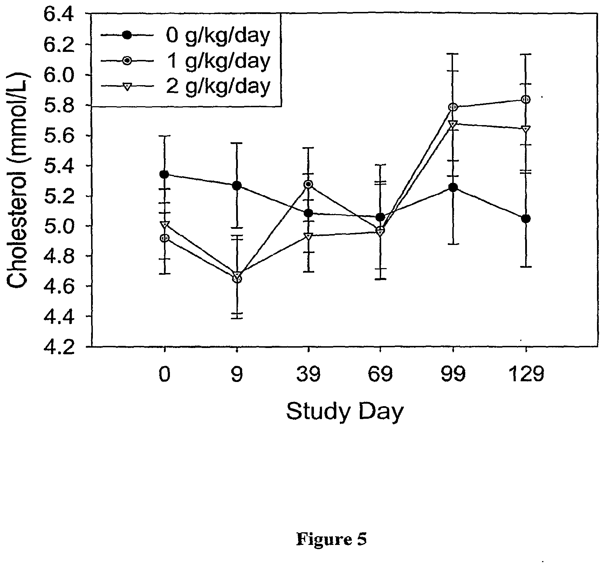

**Figure 5****. Cholesterol Levels.** Cholesterol concentrations were initially lower in the groups receiving MCTs than in the control group, however by study day 99, the treatment groups' cholesterol increased and was higher than the control group's levels. Symbols represent the following treatment groups: dark circles = control group (0 g MCT/kg body weight/day); light circles = 1 g MCT/kg body weight/day; triangles = 2 g MCT/kg body weight/day.

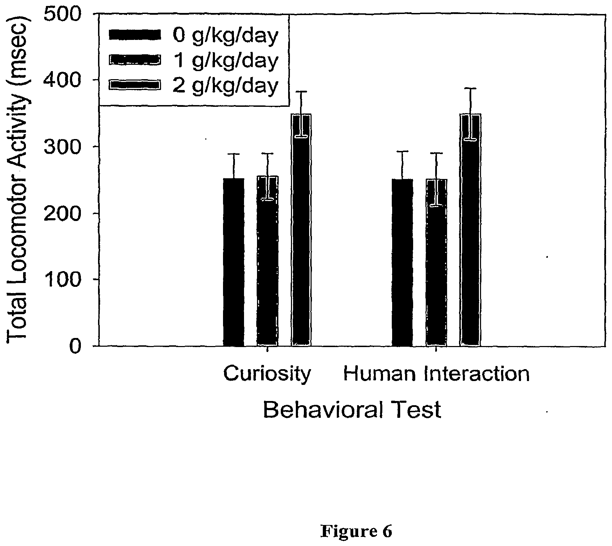

**Figure 6****. The Effect of MCT in the Diet on Behavioral Activity as Reflected by Total Locomotor Activity.** Animals receiving 2 g MCT/kg body weight/day had statistically greater total locomotor activity (TLA) on both curiosity and human activity behavioral tests than the control group or the group receiving the lower dose of MCT.

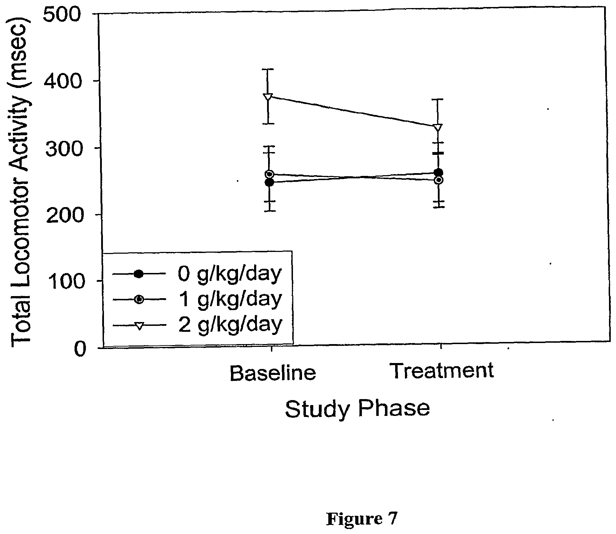

**Figure 7****. Effect of MCT in Diet on Total Locomotor Activity during a Human Interaction Test.** Symbols represent the following treatment groups: dark circles = control group (0 g MCT/kg body weight/day); light circles = 1 g MCT/kg body weight/day; triangles = 2 g MCT/kg body weight/day. The control and lower dose (1 g/kg/day) groups showed little change in total locomotor activity, whereas the 2 g/kg/day group showed a decrease in total locomotor activity from baseline to the treatment phase.

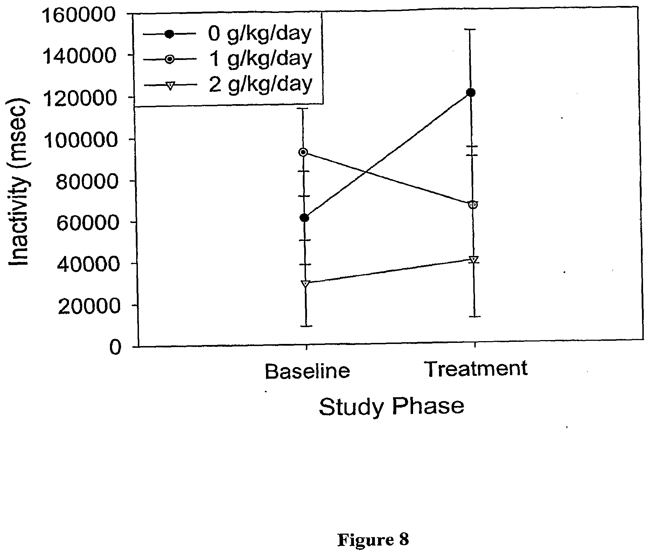

**Figure 8****. The Effect of MCT in the Diet on Results of a Curiosity Test.** The control group showed a large increase in inactivity, whereas the groups receiving MCT at 2 and I g/kg/day group had an increase of much smaller magnitude, and a decrease, respectively, in inactivity between baseline and treatment. Symbols represent the following treatment groups: dark circles = control group (0 g MCT/kg body weight/day); light circles = 1 g MCT/kg body weight/day; triangles = 2 g MCT/kg body weight/day.

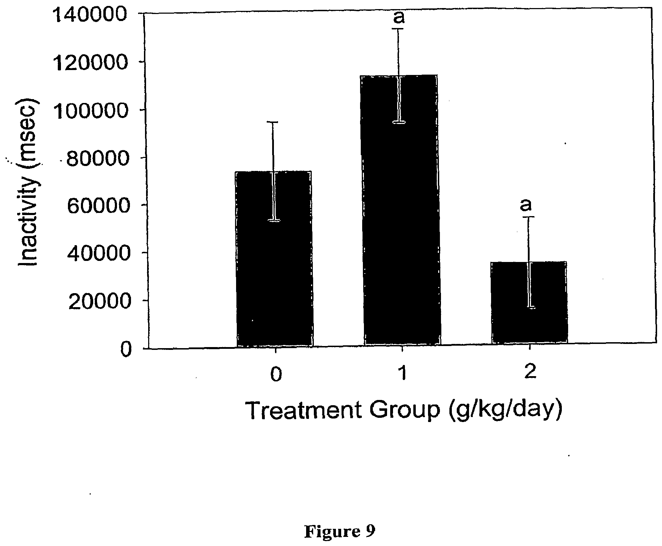

**Figure 9****. Effect of MCT in Diet on Results of a Human Interaction Test.** Each bar represents a study group receiving a specific dosage of MCTs provided with the dietary regimen (0, 1, or 2 g MCT/kg body weight/day). The 2 g/kg/day group had less inactivity than the other groups. Identical letters are indicative of statistically significant differences.

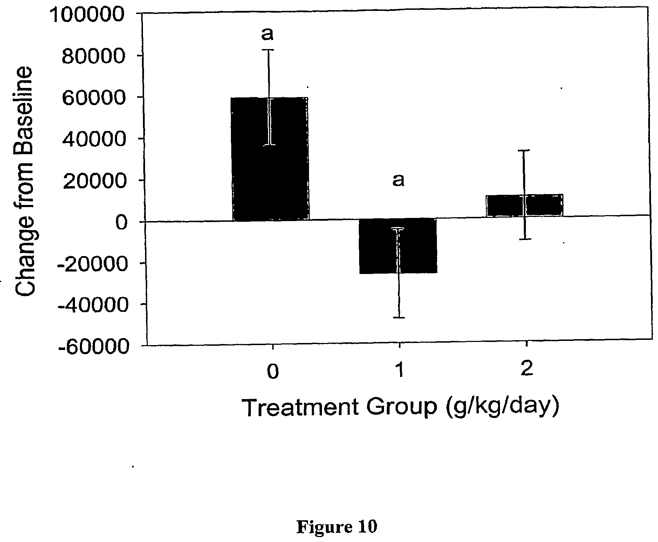

**Figure 10****. Change in Inactivity Levels on the Curiosity Test as a Result of MCT in the Diet.** Animals in the 1 g/kg/day group showed a decrease in inactivity (in msec) on the curiosity test used, whereas the remaining groups showed an increase (control group), or no substantial change (2g/kg/day). The 0 g/kg/day group showed the largest increase in inactivity on this test in the study. Each bar represents a study group receiving a specific dosage of MCTs provided with the dietary regimen (0, 1, or 2 g MCT/kg body weight/day). Identical letters are indicative of statistically significant differences.

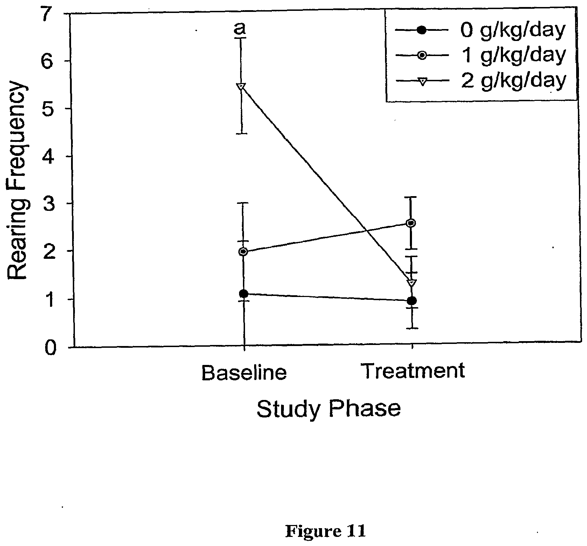

**Figure 11****. The Effect of Dietary MCT on Curiosity Rearing Frequency.** The 2 g/kg/day group showed a large decrease in rearing frequency whereas the remaining groups showed little change. At baseline, the 2 g/kg/day group was significantly different from control, as indicated by the letter (a), but differences with the 1 g/kg/day group were only marginally significant. Symbols represent the following treatment groups: dark circles = control group (0 g MCT/kg body weight/day); light circles = 1 g MCT/kg body weight/day; triangles = 2 g MCT/kg body weight/day.

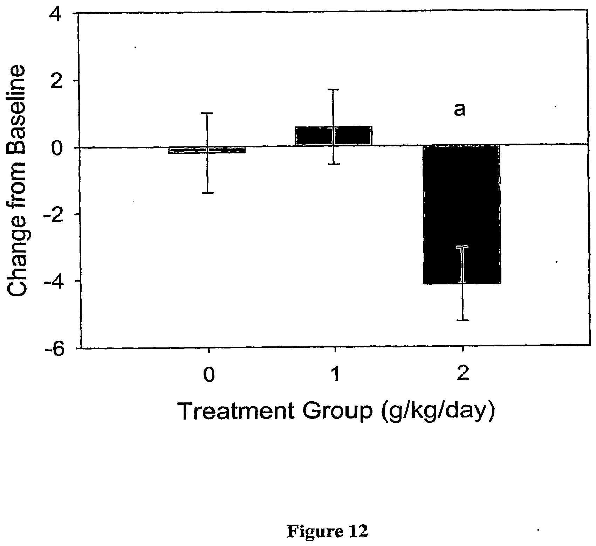

**Figure 12****. Change in Curiosity Rearing Frequency.** The animals receiving MCTs at 2 g/kg/day showed a large decrease in frequency of rearing for curiosity purposes in the study. Each bar represents a study group receiving a specific dosage of MCTs provided with the dietary regimen (0, 1, or 2 g MCT/kg body weight/day) as indicated. The letter (a) indicates a significant differences from the other groups.

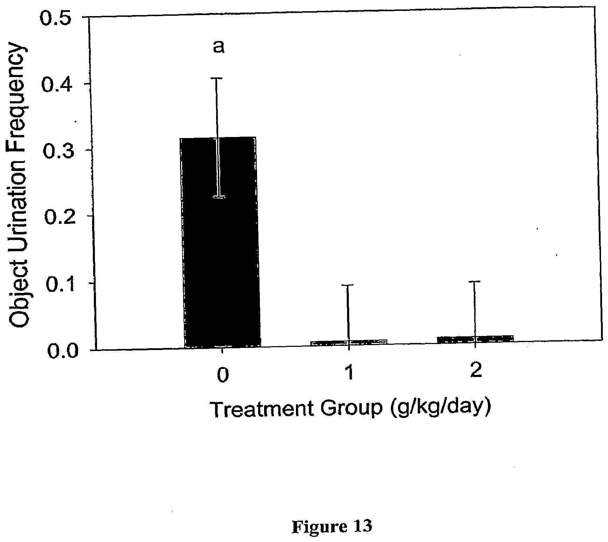

**Figure 13****. Object Urination Frequency as a Function of MCT in the Diet.** The control animals urinated on objects significantly more frequently than animals in groups receiving MCTs at 1 g/kg/day and 2 g/kg/day. Each bar represents a study group receiving a specific dosage of MCTs provided with the dietary regimen (0, 1, or 2 g MCT/kg body weight/day) as indicated. A letter (a) indicates that the group was significantly different from the remaining groups.

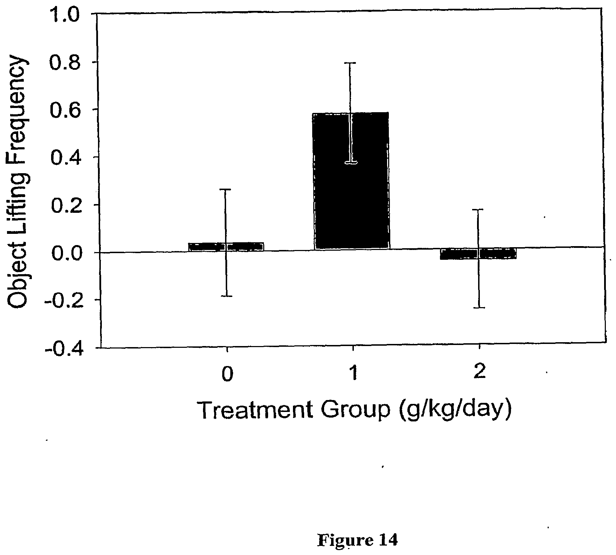

**Figure 14****. Frequency of Lifting Curious Objects as a Function of MCT in the Diet.** Animals in the 1 g/kg/day group picked up objects more frequently than animals in the remaining groups. Each bar represents a study group receiving a specific dosage of MCTs provided with the dietary regimen (0, 1, or 2 g MCT/kg body weight/day).

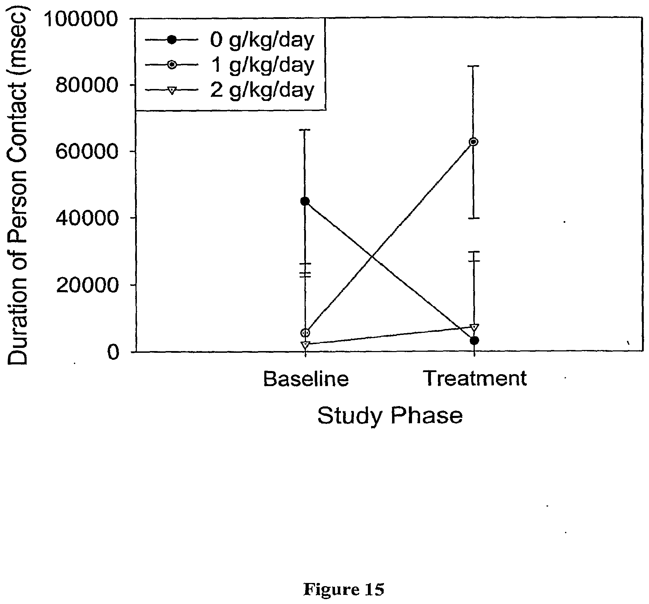

**Figure 15****. The Effect of Dietary MCT on Duration of Person Contact.** Animals receiving MCT at 1 g/kg/day tended to show an increase in duration of person contact during the treatment phase. The control group animals tended to show a decrease in person contact. Symbols represent the following treatment groups: dark circles = control group (0 g MCT/kg body weight/day); light circles = 1 g MCT/kg body weight/day; triangles = 2 g MCT/kg body weight/day.

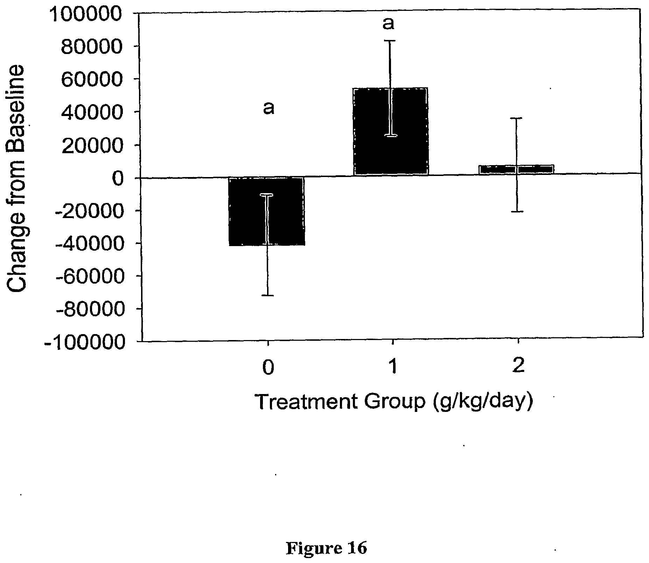

**Figure 16****. Change in the Duration of Person Contact as a Function of Dietary MCT.** Animals receiving MCTs tended to show an increase in person contact duration (in msec) whereas the control animals showed a decrease. Each bar represents a study group receiving a specific dosage of MCTs provided with the dietary regimen (0, 1, or 2 g MCT/kg body weight/day) as indicated. Identical letters indicate statistically significant differences.

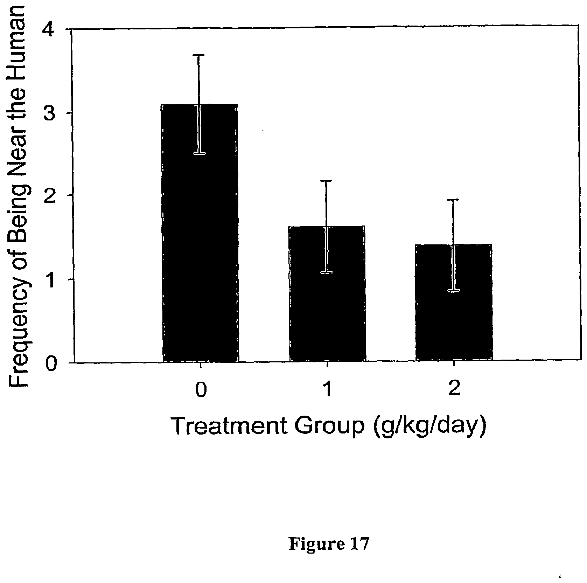

**Figure 17****. Frequency of Being Near the Human as a Function of MCT in the Diet.** The control animals tended to be near the human more frequently than either of the treatment groups. Each bar represents a study group receiving a specific dosage of MCTs provided with the dietary regimen (0, 1, or 2 g MCT/kg body weight/day).

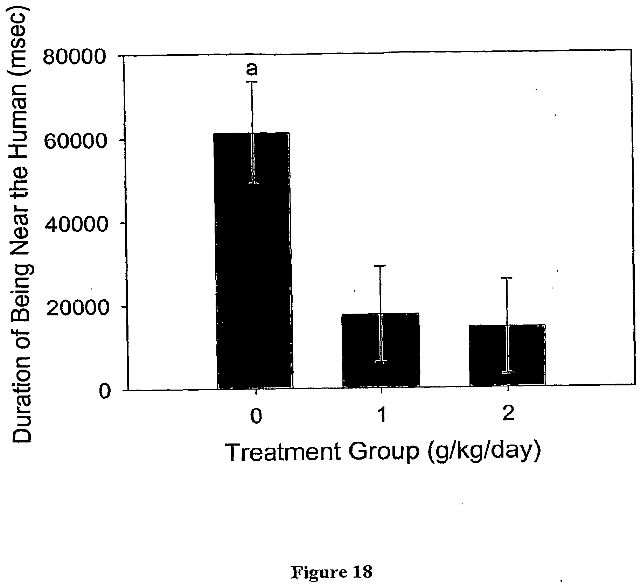

**Figure 18****. Duration of Being Near the Human as a Function of MCT in the Diet.** The control group was near the human longer than either of the treatment groups. As indicated, each bar represents a study group receiving a specific dosage of MCTs provided with the dietary regimen (0, 1, or 2 g MCT/kg body weight/day). The letter (a) indicates that the control group was significantly larger than the remaining groups.

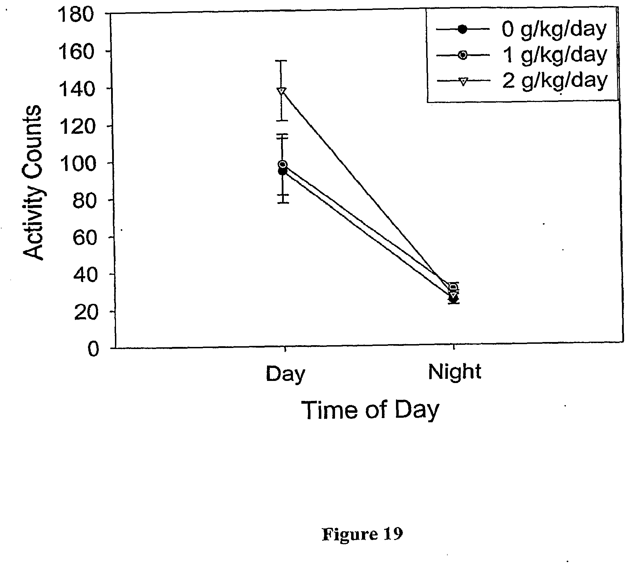

**Figure 19****. The Effect of Dietary MCT on Day and Night Activity.** The group of animals receiving MCT at 2 g/kg/day tended to be more active during the day than the remaining groups - the higher dose of MCT increased daytime activity levels without increasing night time activity. Symbols represent the following treatment groups: dark circles = control group (0 g MCT/kg body weight/day); light circles = 1 g MCT/kg body weight/day; triangles = 2 g MCT/kg body weight/day.

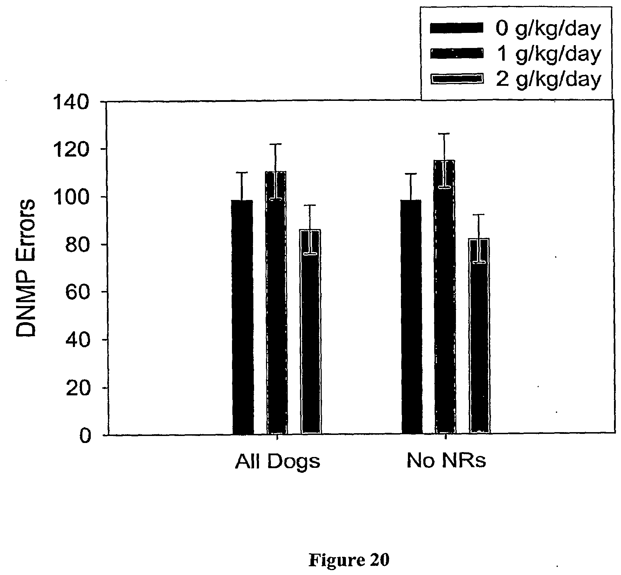

**Figure 20****. The Effect of Dietary MCT on the Number of Errors-to-Criterion on Delayed Non-Match to Position.** The group of animals receiving MCT at 2 g/kg/day tended to make fewer errors when learning the DNMP than either of the control group (0 g/kg/day) or the group receiving the lower dose of MCT (1 g/kg/day). As indicated, each bar represents a study group receiving a specific dosage of MCTs provided with the dietary regimen (0, 1, or 2 g MCT/kg body weight/day). "NRs" indicates

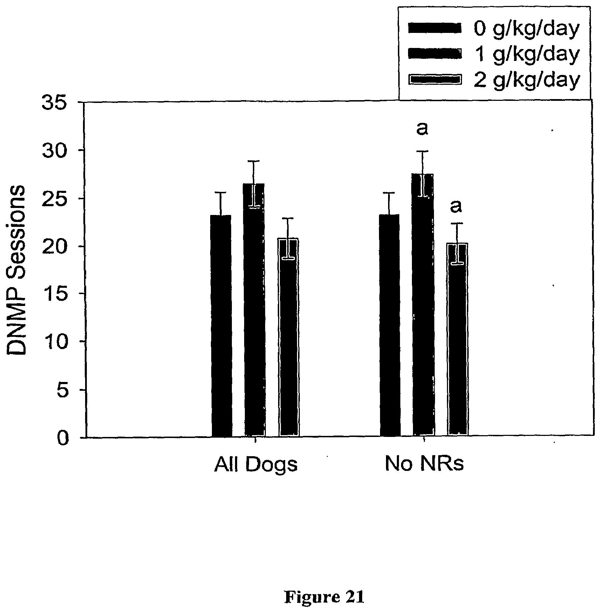

**Figure 21****. The Effect of Dietary MCT on the Number of Sessions-to-Criterion on the Delayed Non-Match to Position.** Animals receiving 2 g/kg/day group tended to require fewer sessions to learn the DNMP. Identical letters indicate statistically significant differences. As indicated, each bar represents a study group receiving a specific dosage of MCTs provided with the dietary regimen (0, 1, or 2 g MCT/kg body weight/day).

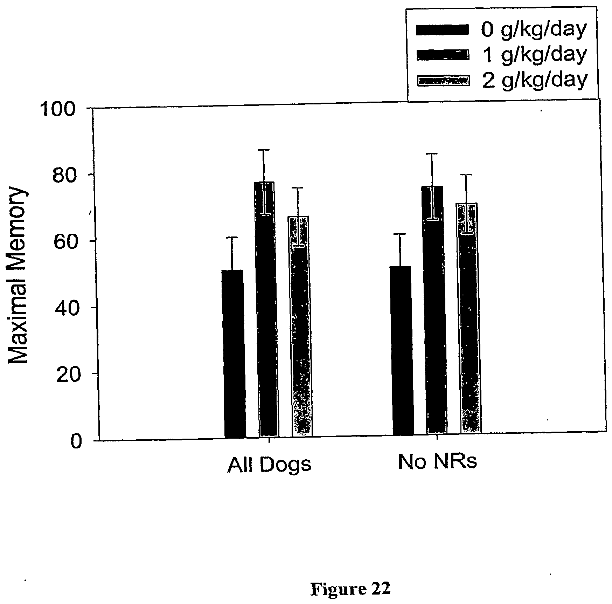

**Figure 22****. The Effect of Dietary MCT on Maximal Memory Scores.** Animals in the groups receiving dietary MCT had larger maximal memory scores than control animals, although the differences did not attain statistical significance. As indicated, each bar represents a study group receiving a specific dosage of MCTs provided with the dietary regimen (0, 1, or 2 g MCT/kg body weight/day).

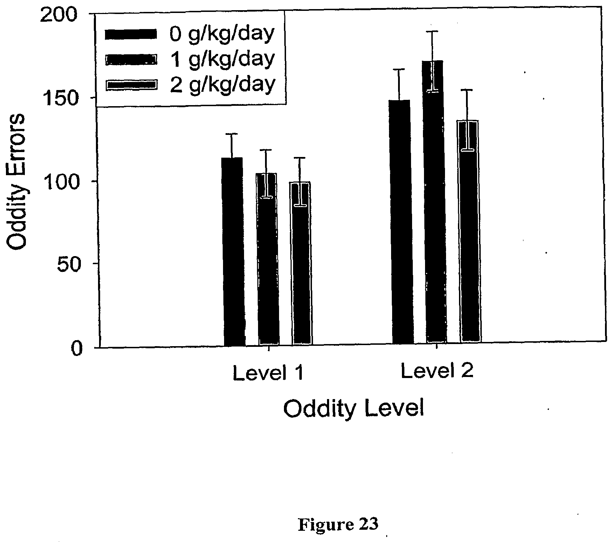

**Figure 23****. The Effect of Dietary MCT on the Number of Errors-to-Criterion on the Oddity Discrimination.** Animals receiving MCTs at 2 g/kg/day made fewer errors to learn each of two oddity tests, although differences did not achieve statistical significance. Each bar represents a study group receiving a specific dosage of MCTs provided with the dietary regimen (0, 1, or 2 g MCT/kg body weight/day).

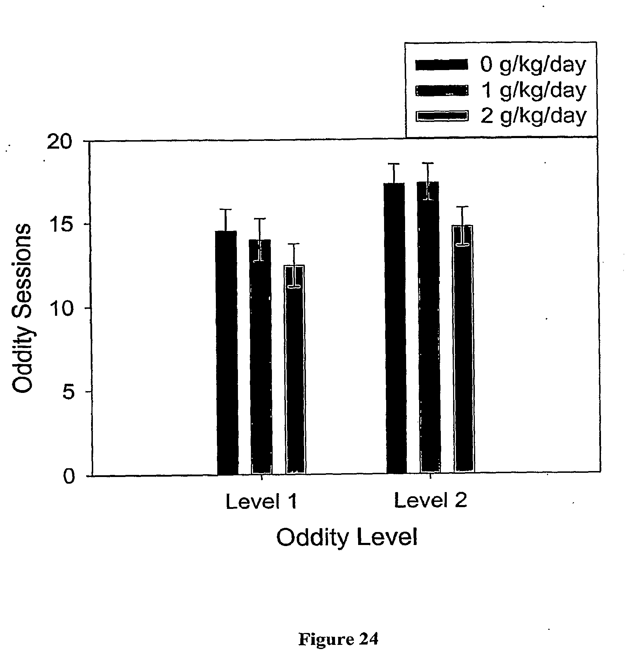

**Figure 24****. The Effect of Dietary MCT on the Number of Sessions-to-Criterion on the Oddity Discrimination.** Animals receiving MCTs at 2 g/kg/day required fewer sessions to learn each of two oddity tests. Each bar represents a study group receiving a specific dosage of MCTs provided with the dietary regimen (0, 1, or 2 g MCT/kg body weight/day). The differences did not reach statistical significance.

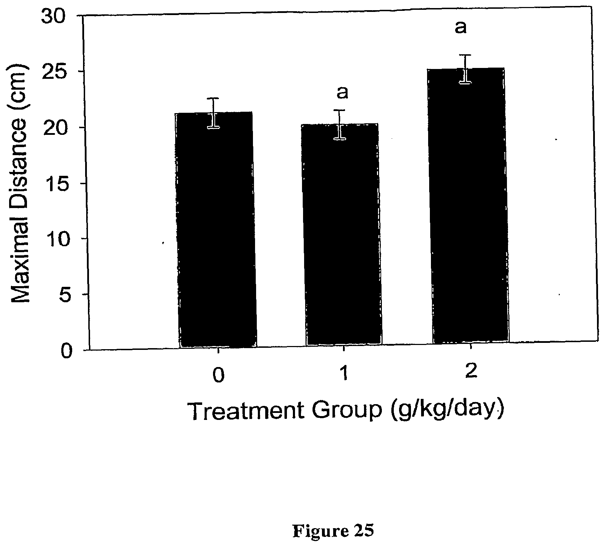

**Figure 25****. The Effect of Dietary MCT on Motor Task Acquisition and Performance.** Animals receiving MCTs at 2 g/kg/day were able to retrieve food from longer distances on the motor task acquisition test. Each bar represents a study group receiving a specific dosage of MCTs provided with the dietary regimen (0, 1, or 2 g MCT/kg body weight/day). Identical letters (a) indicate statistically significant differences.

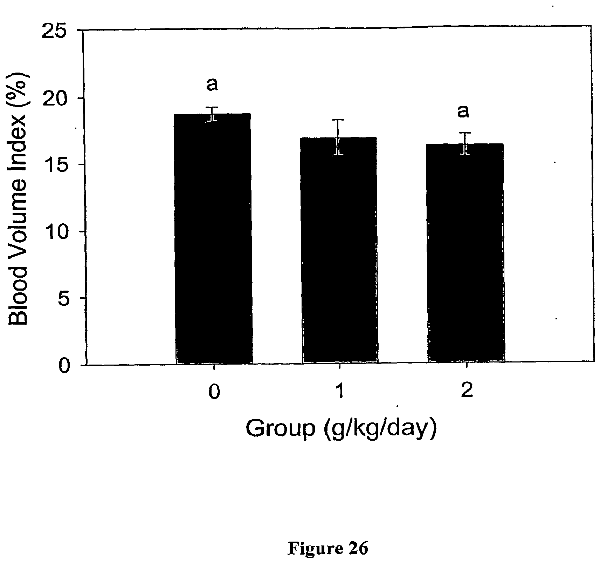

**Figure 26****. Blood Volume Index as a Function of Dietary MCT.** Animals in the group receiving MCT at 2 g/kg/day had smaller Blood Volume (BV) indices than the other groups. Each bar represents a study group receiving a specific dosage of MCTs provided with the dietary regimen (0, 1, or 2 g MCT/kg body weight/day), as indicated. Identical letters indicate marginally significant differences.

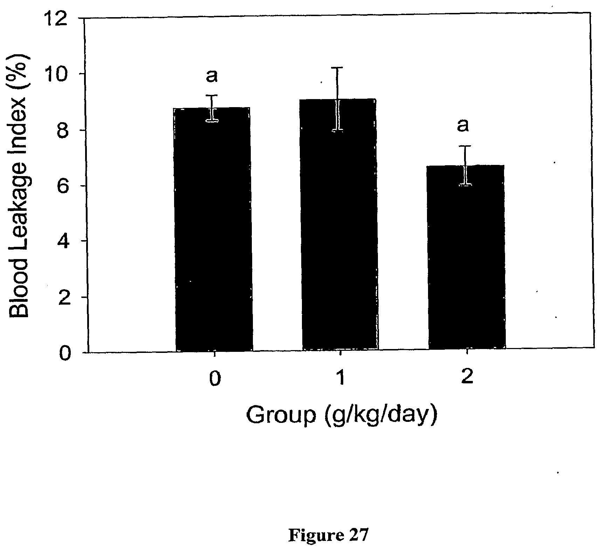

**Figure 27****. Brain Blood Leakage Index as a Function of Dietary MCT.** Animals in the group receiving MCT at 2 g/kg/day had less brain blood leakage than the groups receiving no MCT or lower MCT. Each bar represents a study group receiving a specific dosage of MCTs provided with the dietary regimen (0, 1, or 2 g MCT/kg body weight/day), as indicated. Identical letters indicate statistically significant differences.

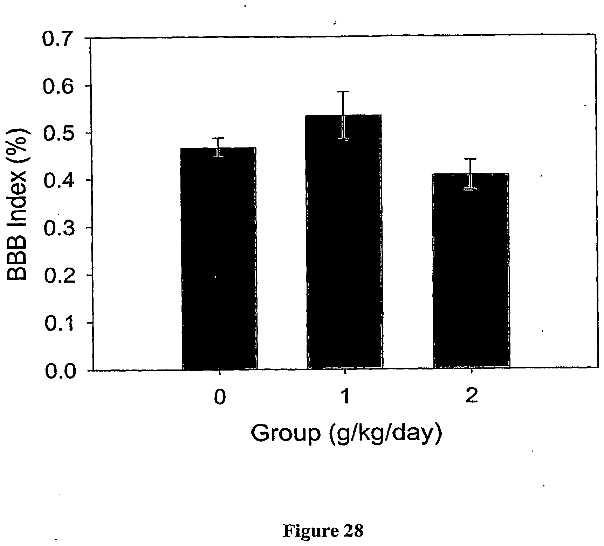

**Figure 28****. Blood Brain Barrier Index as a Function of Dietary MCT.** Animals in the group receiving MCT at 2 g/kg/day tended to have less blood brain barrier (BBB) leakage than the remaining groups. Each bar represents a study group receiving a specific dosage of MCTs provided with the dietary regimen (0, 1, or 2 g MCT/kg body weight/day), as indicated.

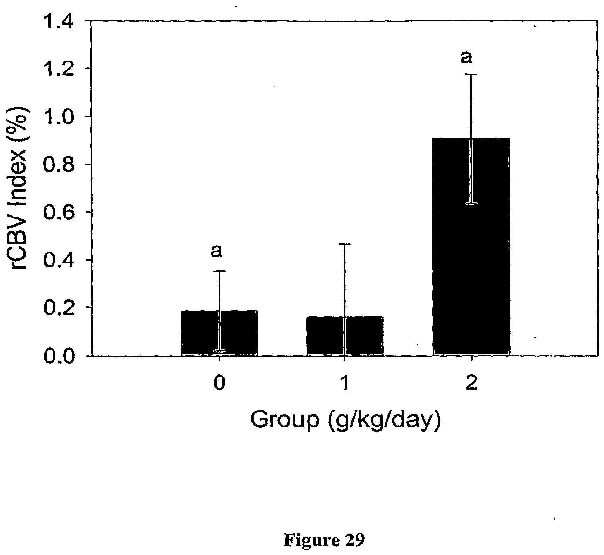

**Figure 29****. Regional Cerebral Volume Index as a Function of Dietary MCT.** Animals in the group receiving MCT at 2 g/kg/day tended to have more regional cerebral blood volume (rCBV) than the control and 1 g/kg/day groups. Each bar represents a study group receiving a specific dosage of MCTs provided with the dietary regimen (0, 1, or 2 g MCT/kg body weight/day), as indicated. Identical letters are indicative of statistical trends.

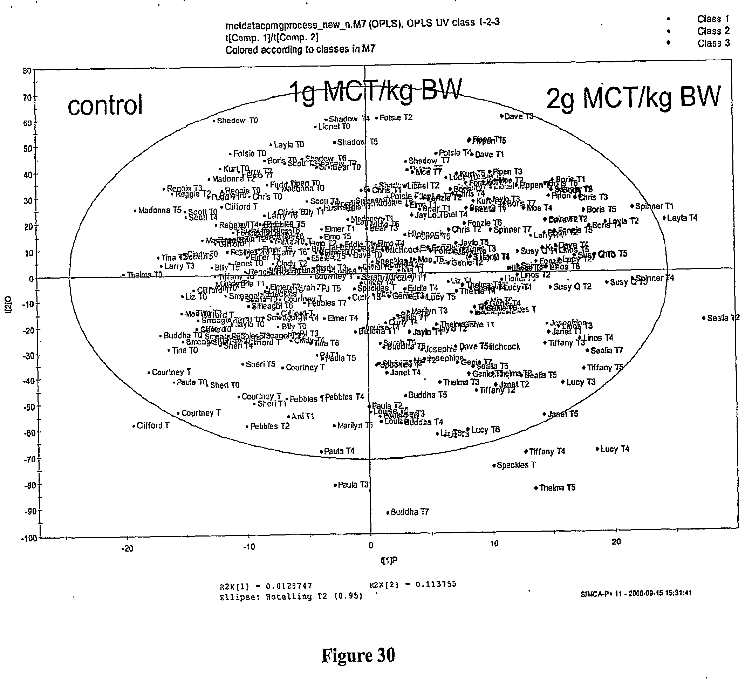

**Figure 30****. Principal Component Analysis For All Treatment Groups.** Principal Component Analysis (PCA) analysis based on metabolomic NMR analysis described in Example 6. Principle components are X-axis and Y-axis are indicated. Data were statistically analyzed and clustered using O-PLS-DA.

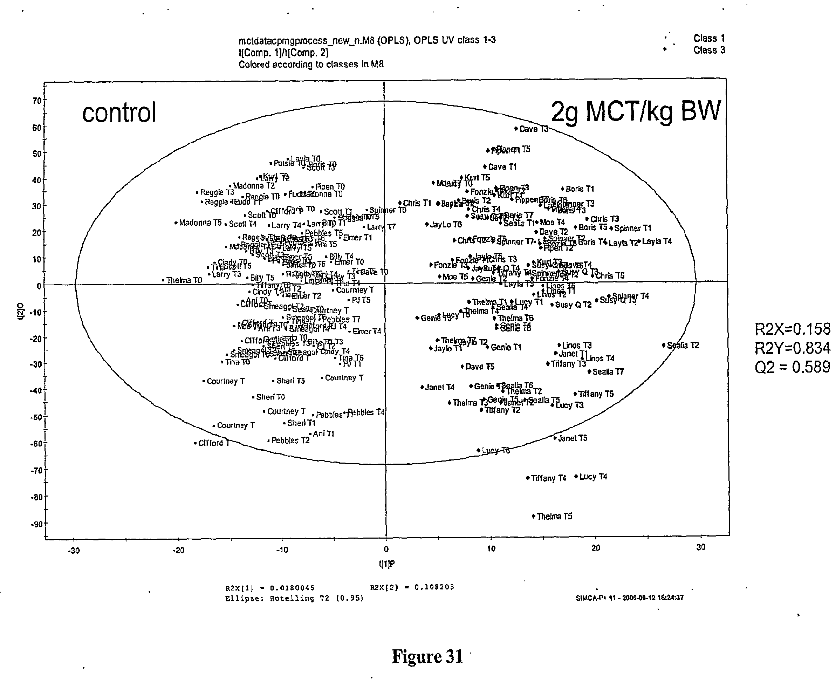

**Figure 31****. Principal Component Analysis For Control vs Dietary MCT at 2 g/kg/day.** PCA analysis shown in Figure 30 with the low-dose (MCT at 1 g/kg/day) data omitted for clarity. Data were statistically analysed and clustered using O-PLS-DA.

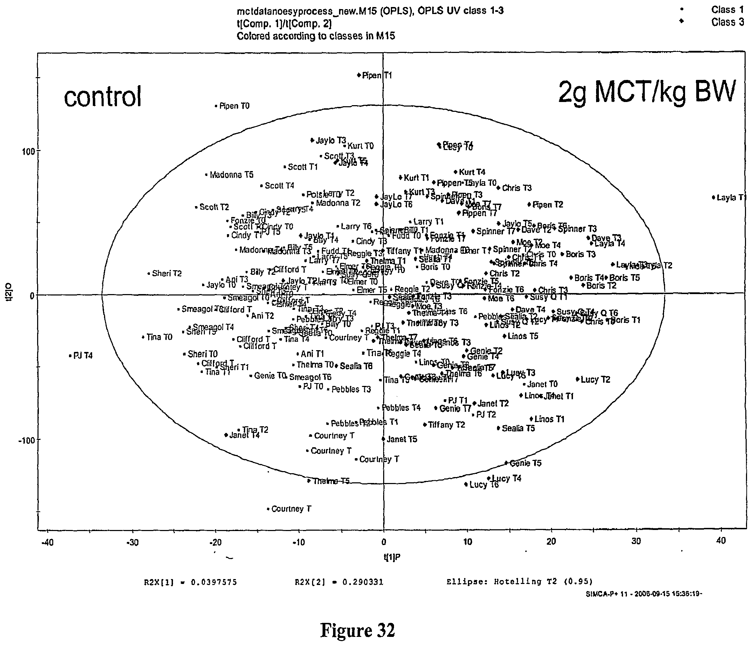

**Figure 32****. Additional PCA for For Control vs Dietary MCT at 2 g/kg/day.** Additional example showing analysis and clustering of metabolomic data using O-PLS-DA.

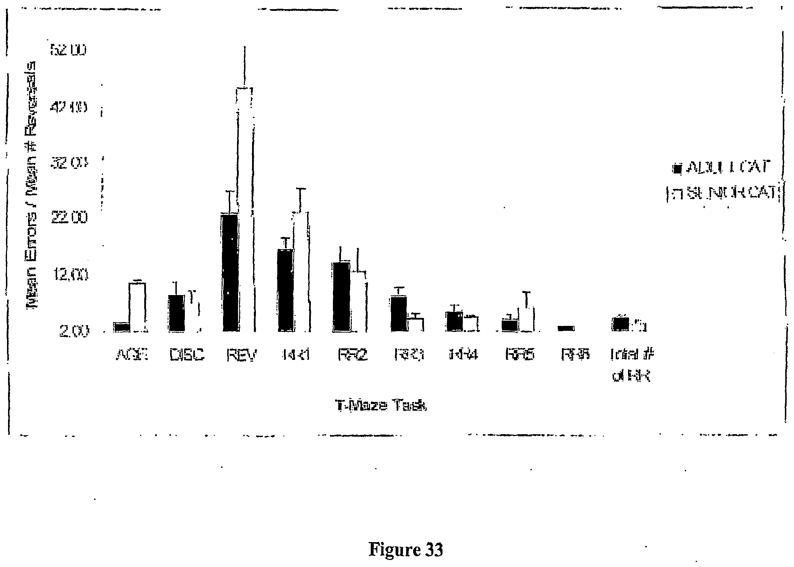

**Figure 33****. Errors Committed by Adult versus Senior cats for the T-Maze task.** The "Senior Cats" on the graph are the combined results for the "Old" and "Senior" cats in the Table 7.1.

### DETAILED DESCRIPTION OF ILLUSTRATIVE EMBODIMENTS

The therapeutic activity of MCTs in humans has been attributed to their conversion to ketone bodies within the liver. Ketone bodies can provide an alternative energy source to supplement the energy deficit in neuronal cells of patients suffering from Alzheimer's disease. It has been discovered in accordance with the present invention that long-term dietary supplementation with MCTs improves cognitive function and results in positive behavioral alterations in aging animals that are not suffering from any known disease. Accordingly, various aspects of this invention are directed to compositions that utilize medium chain triglycerides, used as part of a regular diet, to improve at least one of the following in aging animals: cognitive function, motor performance, cerebrovascular function,

### Definitions:

Various terms relating to the methods and other aspects of the present invention are used throughout the specification and claims. Such terms are to be given their ordinary meaning in the art unless otherwise indicated. Other specifically defined terms are to be construed in a manner consistent with the definition provided herein.

The following abbreviations may be found in the specification and examples:
AD, Alzheimer's Disease;
ALT, Alanine aminotransferase;
ANCOVA, analysis of covarience;
ANOVA, analysis of variance;
AVG, average;
BBB, blood brain barrier;
BBBI, blood brain barrier index;
BHB, beta-hydroxybutyrate;
BLI, blood leakage index;
BVI, blood volume index;
BUN, blood urea nitrogen;
BW, body weight;
CCDS, Canine Cognitive Dysfunction Syndrome;
DNMP, delayed non-match to position;
F, female;
HDL, high-density lipoproteins;
M, male;
MCT, medium chain triglycerides;
MRI, magnetic resonance imaging;
rCBVI, regional cerebral blood volume index;
SEM, standard error of the mean; and
VLDL, very low-density lipoproteins;

"Medium chain triglycerides" or "MCTs" refers to any glycerol molecule ester-linked to three fatty acid molecules, each fatty acid molecule having 5-12 carbons.

The structured lipids of this invention may be prepared by any process known in the art, such as direct esterification, rearrangement, fractionation, transesterification, or the like. For example, the lipids may be prepared by the rearrangement of a vegetable oil such as coconut oil. The length and distribution of the chain length may vary depending on the source oil. For example, MCTs containing 1-10% C6, 30-60% C8, 30-60% C10, 1-10% C10 are commonly derived from palm and coconut oils. MCTs containing greater than about 95% C8 at fatty acids can be made by semi-synthetic esterification of octanoic acid to glycerin. Also useful herein are mixtures comprising MCTs with about 50% total C8 and/or about 50% total C10. Commercial sources for the foregoing MCT compositions are available and known to the skilled artisan. Such MCTs behave similarly and are encompassed within the term MCTs as used herein.

"selective amount" refers to an amount of a compound, material, or composition, as described herein that is effective to achieve a particular biological result. Such results include, but are not limited to, at least one of the following: enhancing cognitive function, improving liver function, increasing daytime activity, improving leaning, improving attention, improving social behavior, improving motor performance, and/or improving cerebrovascular function, particularly in aging or geriatric animals. "effective amount" refers to an amount suitable for to reduce, prevent, or delay a decline in the above qualities, for example, cognitive function or performance, learning rate or ability, problem solving ability, attention span and ability to focus on a task or problem, motor function or performance, social behavior, and the like. Preferably the prevention, reduction, or delay of a such a decline in an individual or population is relative to a cohort - e.g. a control animal or a cohort population that has not received the treatment. Such effective activity may be achieved, for example, by administering the compositions of the present invention to an animal or to a population of animals.

The term "cognitive function" refers to the special, normal, or proper physiologic activity of the brain, including, without limitation, at least one of the following: mental stability, memory/recall abilities, problem solving abilities, reasoning abilities, thinking abilities, judging abilities, capacity for learning, perception, intuition, attention, and awareness. "Enhanced cognitive function" or "improved cognitive function" refers to any improvement in the special, normal, or proper physiologic activity of the brain, including, without limitation, at least one of the following: mental stability, memory/recall abilities, problem solving abilities, reasoning abilities, thinking abilities, judging abilities, capacity for learning, perception, intuition, attention, and awareness, as measured by any means suitable in the art.

"Behavior" is used herein in a broad sense, and refers to anything that an animal does in response or reaction to a given stimulation or set of conditions. "Enhanced behavior" or "improved behavior" refers to any improvement in anything that an animal does in response or reaction to a given stimulation on set of conditions.

"Motor performance" refers to the biological activity of the tissues that affect or produce movement in an animal. Such tissue include without limitation muscles and motor neurons. "Enhanced motor performance" or "improved motor performance" refers to any improvement in the biological activity of the tissues that affect or produce movement in an animal.

"Decline" of any of the foregoing categories or specific types of qualities or functions in an individual (phenotypes) is the generally the opposite of an improvement or enhancement in the quality or function. An "effective amount" (as discussed above) a composition may be an amount required to prevent decline altogether or to substantially prevent decline ("prevent" decline), to reduce the extent or rate of decline ("reduce" decline) over any time course or at any time point, or delay the onset, extent, or progression of a decline ("delay" a decline). Prevention, reduction, or delay of "decline" is frequently a more useful comparative basis when working with nondiseased aging animals. Prevention, reduction, and delay can be considered relative to a control or cohort which does not receive the treatment, for example, the diet or supplement of interest. Prevention, reduction, or delay of either the onset of a detrimental quality or condition, or of the rate of decline in a particular function can be measured and considered on an individual basis, or in some embodiments on a population basis. The net effect of preventing, reducing, or delaying decline is to have less decrease in cognitive, motor, or behavioral functioning per unit time, or at a given end point. In other words, ideally, for an individual or in a population, cognitive, motor, and behavioral functioning is maintained at the highest possible level for the longest possible time. For purposes herein, an individual can be compared to a control individual, group, or population. A population can likewise be compared to an actual individual, to normalized measurements for an individual, or to a group or population as is useful.

"Aging" as used herein means being of advanced age, such that the animal has exceeded 50% of the average lifespan for its particular species. Aging animals are sometimes referred to herein as "aged" or "geriatric" or "elderly." For example, if the average lifespan for a given breed of dog is 10 years, then a dog within that breed greater than 5 years old would be considered geriatric. Healthy aging animals are those with no known diseases, particularly diseases relating to loss of cognitive impairment such as might confound the results. In studies using healthy aging animals, cohort animals are preferably also healthy aging animals, although other healthy animals with suitable cognitive, motor, or behavioral functioning may be suitable for use as comparative specimens. If animals with specific disease diagnoses, or cognitive, motor, or behavioral limitations are used, then the cohort animals should include animals that are similarly diagnosed, or which present with similar indicia of the disease or cognitive, motor, or behavioral limitation.

The present invention relates to dogs and cats.

A "companion animal" is any domesticated animal, and includes, without limitation, cats, dogs, rabbits, guinea pigs, ferrets, hamsters, mice, gerbils, horses, cows, goats, sheep, donkeys, pigs, and the like.

As used herein, the term "food" or "food composition" means a composition that is intended for ingestion by an animal, including a human, and provides nutrition thereto. As used herein, a "food product formulated for human consumption" is any composition specifically intended for ingestion by a human being. "Pet foods" are compositions intended for consumption by pets, preferably by companion animals. A "complete and nutritionally balanced pet food," is one that contains all known required nutrients for the intended recipient or consumer of the food, in appropriate amounts and proportions, based for example on recommendations of recognized authorities in the field of companion animal nutrition. Such foods are therefore capable of serving as a sole source of dietary intake to maintain life or promote production, without the addition of supplemental nutritional sources. Nutritionally balanced pet food compositions are widely known and widely used in the art.

As used herein, a "dietary supplement" is a product that is intended to be ingested in addition to the normal diet of an animal. Dietary supplements may be in any form - e.g. solid, lid, gel, tablets, capsules, powder, and the like. Preferably they are provided in convenient dosage forms. In some embodiments they are provided in bulk consumer packages such as bulk powders or liquids. In other embodiments, supplements are provided in bulk quantities to be included in other food items such as snacks, treats, supplement bars, beverages and the like.

The compositions provided herein and below are generally intended for "long term" consumption, sometimes referred to herein as for 'extended' periods. "Long term" administration as used herein generally refers to periods in excess of two month. Periods of longer than two, three, or four months are preferred. Also preferred are more extended periods that include longer than 5, 6, 7, 8, 9, or 10 months. Periods in excess of 11 months or 1 years are also preferred. Longer terms use extending over 1, 2, 3 years or more are also contemplated herein. In the case of certain aging animals, it is envisioned that the animal would continue consuming the compositions for the remainder of it life on a regular basis. "Regular basis" as used herein refers to
regimens that comprise at least once daily consumption. The skilled artisan will appreciate that the blood level of ketone bodies, or a specific ketone body, achieved may be a valuable measure of dosing frequency. Any frequency, regardless of whether expressly exemplified herein, that allows maintenance of a blood level of the measured compound within acceptable ranges can be considered useful herein. The skilled artisan will appreciate that dosing frequency will be a function of the composition that is being consumed or administered, and some compositions may require more or less frequent administration to maintain a desired blood level of the measured compound (e.g, a ketone body).

As used herein, the term "oral administration" or "orally administering" means that the animal ingests, or a human is directed to feed, or does feed, the animal one or more of the compositions described herein. Wherein a human is directed to feed the composition, such direction may be that which instructs and/or informs the human that use of the composition may and/or will provide the referenced benefit, for example, enhancing cognitive function, improving liver function, increasing daytime activity, improving learning, improving attention, improving social behavior, improving motor performance, and/or improving cerebrovascular function, or preventing, reducing, or delaying a decline in such foregoing functions or qualities. Such direction may be oral direction (*e.g*., through oral instruction from, for example, a physician, veterinarian, or other health professional, or radio or television media (*i.e*., advertisement), or written direction (*e.g*., through written direction from, for example, a physician, veterinarian, or other health professional (*e.g.*, prescriptions), sales professional or organization (*e.g*., through, for example, marketing brochures, pamphlets, or other instructive paraphernalia), written media (*e.g*., internet, electronic mail, or other computer-related media), and/or packaging associated with the composition (*e.g*., a label present on a container holding the composition),

### Compositions:

In several of its various aspects, the invention provides compositions comprising medium chain triglycerides (MCTs) in an amount effective for the prevention, reduction, or delay of decline of cognitive function, motor function, and/or cerebrevascular function in a dog or a cat. In some embodiments the animals are predisposed to undergoing some decrease or diminishment of cognitive function, motor function or behavioral abilities. In other embodiments, the animals of interest are aging or geriatric animals as defined herein. Preferably, the animals are otherwise healthy. The MCTs can be present in the composition as an ingredient or additive. In preferred embodiments the composition comprises at least one source MCTs.

In one aspect, provided are compositions comprising medium chain triglycerides (MCTs), in an amount effective for improving, or preventing decline of one or more of cognitive function, motor performance, cerebrovascular function, or behavior in an aging mammal. The aging or geriatric mammal will have reached at least about 50% of its life expectancy. The compositions increase circulating concentration of at least one ketone body in the mammal. The MCTs are are composed of fatty acids, each independently having 5-12 carbons, esterified to a glycerol backbone.

In various embodiments, the compositions comprise MCTs with greater than about 95% of the ; C8 fatty acids. In one embodiment the remaining fatty acids are preferably or even exclusively C6 or C10 fatty acids.
The mammal is a dog or cat.

In one embodiment, the mammal is a healthy aging mammal, as defined herein above. In such embodiments, the animal will not be known to have overt signs or substantial symptoms or indicia of cognitive impairment, as determined by a skilled artisan. Although the animal may have other health issues, even age-related health issues, they are preferably of such character as to not substantially impact the cognitive, motor, or behavioral functioning of the animal. Thus, the skilled artisan will appreciate that it may be impossible to classify an aging or geriatric animal as completely "healthy" -however, it is not necessary to do so to practice the methods and compositions provided herein. In other embodiments, the aging animal is specifically understood to have age-related cognitive impairment, whether determined by formal diagnosis, or by its evidencing hallmarks of cognitive or motor impairments or behavioral indicia of such impairment or the like. In one embodiment, the mammal has a phenotype associated with age-related cognitive impairment, for example the animal has one or more of the following phenotypic expressions of cognitive, motor, or behavioral difficulties associated with age. For example, decreased ability to recall, short-term memory loss, decreased learning rate, decreased capacity for learning, decreased problem solving skills, decreased attention span, decreased motor performance, increased confusion, or dementia, as compared to a control mammal not having the phenotype.

In one embodiment, the compositions of the invention are food compositions, such as pet foods. In certain embodiments, The composition is a food composition, further comprising in addition to the MCTs, about 15-50% protein, about 5-40% fat, about 15-60% carbohydrate, 5-10% ash content, each on a dry weight basis, and having a moisture content of about 5-20%. In certain embodiments, the foods are intended to supply complete necessary dietary requirements. Also provided are compositions that are useful as snacks, pet treats (*e.g*., biscuits), nutrition bars, and other forms for food products or dietary supplements, including tablets, capsules, gels, pastes, emulsions, caplets, and the like as discussed below. Optionally, the food compositions can be a dry composition (for example, kibble for pet food), semi-moist composition, wet composition, or any mixture thereof.

Further described are the compositions as food products formulated specifically for human consumption. These will include foods and nutrients intended to supply necessary dietary requirements of a human being as well as other human dietary supplements. it is described that the food products formulated for human. consumption arc complete and nutritionally balanced, while in others they are intended as dietary supplements to be used in connection with a well-balanced or formulated diet.

In another embodiment, the composition is a food supplement, such as a gravy, drinking water, beverage, liquid concentrate, gel, yogurt, powder, granule, paste, suspension, chew, morsel, treat, snack, pellet, pill, capsule, tablet, or any other delivery form. The dietary supplements can be especially formulated for consumption by a dog or a cat.

In one embodiment, the dietary supplement can comprise a relatively concentrated dose of MCTs such that the supplement can be administered to the animal in small amounts, or can be diluted before administration to an animal. In some embodiments, the dietary supplement or other MCT-containing composition may require admixing with water or the like prior to administration to the animal, for example to adjust the dose, to make it more palatable, or to allow for more frequent administration in smaller doses.

The MCT-containing compositions may be refrigerated or frozen. The MCTs may be pre-blended with the other components of the composition to provide the beneficial amounts needed, may be emulsified, coated onto a pet food composition, dietary supplement, or food product formulated for human consumption, or may be added to a composition prior to consuming it or offering it to an animal, for example, using a powder or a mix.

In one embodiment, the compositions comprise MCTs in an amount effective to enhance cognitive function a dog or a cat to which the composition has been administered. For pet foods the amount of MCTs as a percentage of the composition is in the range of about 1% to about 30% of the composition on a dry matter basis, although a lesser or greater percentage can be supplied. In various embodiments, the amount is about 1.0%, 1.5%, 2.0%, 2.5%, 3.0%, 3.5%, 4.0%, 4.5%, 5.0%, 5.5%, 6%, 6.5%, 7%, 7.5%, 8%, 8.5%, 9%, 9.5%, 10%, 10.5%, 11%, 11.5%, 12%, 12.5%,13%, 13.5%, 14%, 14.5%, 15%, 15.5%, 16%, 16.5%, 17%, 17.5%, 18%, 18.5%, 19%, 19.5%, 20%, 20.5%, 21%, 21.5%, 22%, 22.5%, 23%, 23.5%, 24%, 24.5%, 25.5%, 26%, 26.5%, 27%, 27.5%, 28%, 28.5%, 29%, 29.5%. 30%, or more, of the composition on a dry weight basis. Dietary supplements may be formulated to contain several fold higher concentrations of MCTs, to be amenable for administration to an animal in the form of a tablet, capsule, liquid concentrated, or other similar dosage form, or to be diluted before administrations, such as by dilution in water, spraying or sprinkling onto a pet food, and other similar modes of administration. For a dietary supplement, MCTs alone may be administered directly to the animal or applied directly to the animal's regular food. Dietary supplement formulations in various embodiments contain about 30% to about 100% MCTs, although lesser amounts may also used.

Sources of the MCTs include any suitable source, synthetic or natural. Examples of natural sources of MCT include plant sources such as coconuts and coconut oil, palm kernels and palm kernel oils, and animal sources such as milk from any of a variety of species.

In various embodiments, the compositions optionally comprise supplementary substances such as minerals, vitamins, salts, condiments, colorants, and preservatives. Non-limiting examples of supplementary minerals include calcium, phosphorous, potassium, sodium, iron, chloride, boron, copper, zinc, magnesium, manganese, iodine, selenium, and the like. Non-limiting examples of supplementary vitamins include vitamin A, any of the B vitamins, vitamin C, vitamin D, vitamin E, and vitamin K, including various salts, esters, or other derivatives of the foregoing. Additional dietary supplements may also be included, for example, any form of niacin, pantothenic acid, inulin, folic acid, biotin, amino acids, and the like, as well as salts and derivatives thereof. In addition, the compositions may comprise beneficial long chain polyunsaturated fatty acids such as the (n-3) and/or (n-6) fatty acids, arachidonic acid, eicosapentaenoic acid, docosapentaenoic acid, and docosahexaenoic acid, as well combinations thereof.

The compositions provided herein optionally comprise one or more supplementary substances that promote or sustain general neurologic health, or further enhance cognitive function. Such substances include, for example, choline, phosphatidylserine, alpha-lipoic acid, CoQ10, acetyl-L-carnitine, and herbal components or extracts containing for example, one or more components from such plants as *Ginko biloba, Bacopa monniera, Convolvulus pluricaulis,* and/or *Leucojum aestivum.*

In various embodiments, the pet food or dietary supplement compositions provided herein preferably comprise, on a dry weight basis, from about 15% to about 50% crude protein. The crude protein material comprise one or more proteins from any source whether animal, plant, or other. For example, vegetable proteins such as soybean, cottonseed, and peanut are suitable for use herein. Animal proteins such as casein, albumin, and meat protein, including pork, lamb, equine, poultry, fish, or mixtures thereof are useful.

The compositions may further comprise, on a dry weight basis, from about 5% to about 40% fat. The compositions may further comprise a source of carbohydrate. The compositions typically comprise from about 15% to about 60% carbohydrate, on a dry weight basis. Examples of such carbohydrates include grains or cereals such as rice, corn, sorghum, alfalfa, barley, soybeans, canola, oats, wheat, or mixtures thereof. The compositions also optionally comprise other components that comprise carbohydrates such as dried whey and other dairy products or by-products.

In certain embodiments, the compositions also comprise at least one fiber source. Any of a variety of soluble or insoluble fibers suitable for use in foods or feeds may be utilized, and such will be known to those of ordinary skill in the art. Presently preferred fiber sources include beet pulp (from sugar beet), gum arabic, gum talha, psyllium, rice bran, carob bean gum, citrus pulp, pectin, fructooligosaccharide additional to the short chain oligofructose, mannanoligofructose, soy fiber, arabinogalactan, galactooligosaccharide, arabinoxylan, or mixtures thereof. Alternatively, the fiber source can be a fermentable fiber. Fermentable fiber has previously been described to provide a benefit to the immune system of a companion animal. Fermentable fiber or other compositions known to those of skill in the art which provide a prebiotic composition to enhance the growth of probiotic microorganisms within the intestine may also be incorporated into the composition to aid in the enhancement of the benefit provided by the present invention to the immune system of an animal. Additionally, probiotic microorganisms, such as *Lactobacillus* or *Bifidobacterium* species, for example, may be added to the composition.

In a one embodiment, the composition is a complete and nutritionally balanced pet food. In this context, the pet food may be a wet food, a dry food, or a food of intermediate moisture content, as would be recognized by those skilled in the art of pet food formulation and manufacturing. "Wet food" describes pet food that is typically sold in cans or foil bags, and has a moisture content typically in the range of about 70% to about 90%. "Dry food" describes pet food which is of a similar composition to wet food, but contains a limited moisture content, typically in the range of about 5% to about 15%, and therefore is presented, for example, as small biscuit-like kibbles. The compositions and dietary supplements may be specially formulated for adult animals, or for older or young animals, for example, formulations specifically adapted for puppies, kittens, or "senior" are known in the art and commercially available. In general, specialized formulations will comprise energy and nutritional requirements appropriate for animals at specific stages of development or age. Formulations for overweight animals, or animals with other health issues are also known in the art and are suitable for use herein

In certain embodiments, the compositions provide a complete and balanced food (for example, as described in National Research Council, 1985, Nutritional Requirements for Dogs, National Academy Press, Washington D.C., or Association of American Feed Control Officials, Official Publication 1996). In other embodiments, the compositions are intended to be used in conjunction with such foods. That is, compositions comprising MCTs according to certain embodiments provided herein are used in conjunction with high-quality commercial food. As used herein, "high-quality commercial food" refers to a diet manufactured to produce the digestibility of the key nutrients of 80% or more, as set forth in, for example, the recommendations of the National Research Council above for dogs. Similar high nutrient standards would be used for other animals.

The skilled artisan will understand how to determine the appropriate amount of MCTs, to be added to a given composition. Such factors that may be taken into account include the type of composition (*e.g*., pet food composition, dietary supplement, or food product formulated for human consumption), the average consumption of specific types of compositions by different animals, the intended or required dose of MCTs, the palatability and acceptability of the final product for the intended recipient or consumer, the manufacturing conditions under which the composition is prepared, the convenience for the purchaser, and packaging considerations. Preferably, the concentrations of MCTs to be added to the composition are calculated on the basis of the energy and nutrient requirements of the animal. The MCTs can be added at any time during the manufacture and/or processing of the composition whether as part of a formulation of a pet food composition, dietary supplement, or food product for human consumption, or as a coating or additive to any of the foregoing.

The skilled artisan will appreciate that the compositions provided herein can be formulated and manufactured according to any suitable methods known in the art, for example, the methods described in Waltham Book of Dog and Cat Nutrition, Ed. ATB Ednoy, Chapter by A. Rainbird, entitled "A Balanced Diet" in pages 57 to 74, Pergamon Press Oxford.

### Methods: (not according to the invention)

Further described are methods for improving, and/or preventing, reducing or delaying a decline in, one or more of cognitive function, motor function and behavior in an animal, particularly a geriatric animal, comprising administering to the animal a composition comprising MCTs in an amount effective to improve, and/or prevent, reduce, or delay decline in cognitive function and behavior in the animal.

Thus, methods are described for preventing, reducing, or delaying decline in at least one of cognitive function, motor function, cerebrovascular function, or behavior in an mammal. It is described that : the mammal is an aging or geriatric animal. The methods comprise the steps of:
(a) identifying a mammal, such as an aging mammal, having, or at risk of, decline in at least one of cognitive function, motor function, cerebrovascular function, or behavior; and
(b) administering to the mammal on an extended regular basis a composition comprising medium chain triglycerides (MCTs) in an amount effective to prevent, reduce, or delay decline in at least one of cognitive function, motor function, cerebrovascular function, or behavior in the mammal. It is described that the composition increases the circulating concentration of at least one ketone body in the mammal. As with the compositions provided above, the MCTs used herein are composed of fatty acids, each independently having 5-12 carbons, esterified to the glycerol backbone.

It is described that greater than about 95% of the fatty acids are 8 carbons in length. The remaining fatty acids are 6-carbon or 10-carbon fatty acids in some embodiments. In other embodiments, greater than at least or about 30, 40, or 50% of fatty acids are C8 and/or greater than at least or about 30,40, or 50% of fatty acids are C10. In once embodiment about 50% of the are C8 and about 50% of fatty acids are C10.

It is described that the method further comprises the step of monitoring the concentration of at least one ketone body in the mammal. The skilled artisan will appreciate that there arc ways known to measure blood or plasma concentrations of ketone bodies collectively, or individually. All such methods are suitable for use herein in monitoring the ketone concentration in the mammal.

It is described that the administered composition comprises MCTs such that the amount of each of β-hydroxybutyrate, acetoacetate and acetone is raised in the blood of the mammal, particularly relative to a mammal not receiving the composition.

It is described that the methods comprise an administration step wherein the composition comprises MCTs in an amount effective for lowering the amount in the blood of the mammal of one or more of alanine, branched chain amino acids, total lipoproteins, unsaturated fatty acids, or VLDL, or wherein the amount of each of alanine, branched chain amino acids, total lipoproteins, unsaturated fatty acids, and VLDL is lowered in blood of the mammal.

It is described that the composition administered comprises MCTs in an amount effective for raising an amount in the blood of the mammal of one or more of glutamine, phenylalanine, HDL, or citrate, while in yet other embodiments, the amount of each of glutamine, phenylalanine, HDL, and citrate is raised in the blood of the mammal.

It is described that, the methods comprise an administration step wherein the composition comprises MCTs in an amount effective for lowering the amount of each of alanine, branched chain amino acids, total lipoproteins, unsaturated fatty acids, and VLDL, in addition to raising the amount of each of glutamine, phenylalanine, HDL, and citrate in the blood of the mammal.

It is described that the administered composition comprises MCTs in an amount effective for improving blood flow to the brain, or for improving the integrity of the blood brain barrier, or both. Such improvements can be measured with an individual over time, or relative to a control not receiving the composition.

It is described that the composition administered is a pet food, dietary supplement, or a food product formulated for human consumption, and in numerous embodiments, the mammal is a companion animal. the companion animal is a cat or dog.

The composition administered comprises at least about 1% to about 30% MCTs on a dry weight basis in various applications of the methods. It is described that the administering step is on a regular basis comprising at least once daily. It is described that
the composition is administered as part of a daily dietary regimen for at least about one week. A duration of two, three or even four weeks is also used. Administering the compositions for one to three months, or four months is exemplified herein. In other embodiments, it is contemplated that administration will be extended for 4, 5, 6, 7, 8, 9, 10, 11 or even 12 months. In yet longer applications, administration periods extending 1, 2, 3, or more years are anticipated. It is described that it may be useful to at least periodically monitor the animal for ketoacidosis and the like, however, there is no evidence that the compositions or methods provided herein will result in ketoacidosis even after prolonged administration It is described that the administration of the compositions is maintained for the remainder of the animal's life (for example, the second half of the life expectancy for a animal that has just recently attained aged or geriatric status, as defined herein).

It is described that the composition is administered as part of a daily dietary regimen for at least about once week, about three months, or about one year at a minimum.

It is described that the composition administered comprises MCTs in an amount affective for lowering blood urea nitrogen or decreasing protein degradation. In another, the composition comprises MCTs in an amount effective for lowering the amount or activity of the enzyme, alanine aminotransferase.

It is described that the methods described comprise an administration step wherein the composition comprises MCTs in an amount effective for improving social behaviors of a companion animal. Such improvement can comprise interaction with its own or other species, such as a human.

It is described that the composition is a pet food composition, dietary supplement, or food product formulated for human consumption as exemplified herein, Animals can include any domesticated or companion animals as described above, or can include humans except as would result in construing the invention to encompass a prior method or composition known to those of skill. It is described that the animal is a companion animal such as a dog or cat. Further it is described that the animal is a human.

The compositions can be administered to the animal by any of a variety of alternative routes of administration. Such routes include, without limitation, oral, intranasal, intravenous, intramuscular, intragastric, transpyloric, subcutaneous, rectal, and the like. Preferably, the compositions are administered orally.

Administration can be on an as-needed or as-desired basis, for example, once-monthly, once-weekly, daily, or more than once daily. Similarly, administration can be every other day, week, or month, every third day, week, or month, every fourth day, week, or month, and the like. Administration can be multiple times per day. When utilized as a supplement to ordinary dietetic requirements, the composition may be administered directly to the animal or otherwise contacted with or admixed with daily feed or food. When utilized as a daily feed or food, administration will be well known to those of ordinary skill.

Administration can also be carried out on a regular basis, for example, as part of a diet regimen in the animal. A diet regimen may comprise causing the regular ingestion by the animal of a composition comprising MCTs in an amount effective to enhance cognitive function and behavior in the animal. Regular ingestion can be once a day, or two, three, four, or more times per day, on a daily or weekly basis. Similarly, regular administration can be every other day or week, every third day or week, every fourth day or week, every fifth day or week, or every sixth day or week, and in such a dietary regimen, administration can be multiple times per day. The goal of regular administration is to provide the animal with the preferred daily dose of MCTs, as exemplified herein.

The daily dose of MCTs can be measured in terms of grams of MCTs per kg of body weight (BW) of the animal. The daily dose of MCTs can range from about 0.01g/kg to about 10.0g/kg BW of the animal. Preferably, the daily dose of MCTs is from about 0.1g/kg to about 5 g/kg BW of the animal. More preferably, the daily dose of MCTs is from about 0.5 g/kg to about 3 g/kg of the animal. Still more preferably, the daily dose of MCTs is from about 1 g/kg to about 2 g/kg of the animal.

It is described that administration of the compositions comprising MCTs, including administration as part of a diet regimen, can span a period of time ranging from gestation through the entire life of the animal. Preferably, the compositions comprising MCTs are administered to geriatric animals. Although different species of animals reach advanced age at different rates, those of skill in the art will understand and appreciate when a given species has reached an advanced age. Determination of the appropriate age for a given animal in which to administer compositions comprising MCTs can routinely be accomplished by those of skill in the art

It is described that methods are described for improving, or for preventing, reducing, or delaying decline in, at least once of cognitive function, motor function, cerebrovascular function, or behavior in an aging mammal. The methods generally comprise the steps of:
(a) identifying an aging mammal not having an age-related cognitive impairment disease; (also sometimes referred to herein as a healthy aging mammal) and
(b) administering to the mammal, on an extended regular basis as defined herein, a composition comprising medium chain triglycerides (MCTs) in an amount effective to improve, or prevent, reduce, or delay decline in at least one of cognitive function, motor function, cerebrovascular function, or behavior in the mammal;
   wherein said composition increases the circulating concentration of at least one ketone body in the mammal;
(c) measuring the concentration of at least one ketone body, and at least one of cognitive function, motor function, cerebrovascular function, or behavior in the mammal at least periodically for the duration of the administering step;
(d) comparing the at least one ketone body concentration and the measure of cognitive function, motor fraction, cerebrovascular function, or behavior to that of a control animal not receiving the administered composition;
(e) correlating the ketone body concentration with the measure of cognitive function, motor function, cerebrovascular function, or behavior thereby establishing the prevention, reduction, or delay of the decline of at least one of cognitive function, motor function, cerebrovascular function, or behavior as a result of the administration of the composition.

In the methods described the MCTs are composed of fatty acids, each independently having 5-12 carbons, esterified to the glycerol backbone.

Greater than about 95% of the fatty acids are 8 carbons in length. The remaining fatty acids are 6-carbon or 10-carbon fatty acids : It is described that the administered composition comprises MCTs such that the amount of each of β-hydroxybutyrate, acetoacetate and acetone is raised in the blood of the mammal, particularly relative to a mammal not receiving the composition.

It is described that the methods comprise an administration step wherein the composition comprises MCTs in an amount effective for lowering the amount in the blood of the mammal of one or more of alanine, branched chain amino acids, total lipoproteins, unsaturated fatty acids, or VLDL, or wherein the amount of each of alanine, branched chain amino acids, total lipoproteins, unsaturated fatty acids, and VLDL is lowered in blood of the mammal.

It is described that the composition administered comprises MCTs in an amount effective for raising an amount in the blood of the mammal of one or more of glutamine, phenylalanine, HDL, or citrate, while in yet other embodiments, the amount of each of glutamine, phenylalanine, HDL, and citrate is raised in the blood of the mammal.

It is described that the methods comprise an administration step wherein the composition comprises MCTs in an amount effective for lowering the amount of each of alanine, branched chain amino acids, total lipoproteins, unsaturated fatty acids, and VLDL, in addition to raising the amount of each of glutamine, phenylalanine, HDL, and citrate in the blood of the mammal.

It is described that the administered composition comprises MCTs in an amount effective for improving blood flow to the brain, or for improving the integrity of the blood brain barrier, or both. Such improvements in blood flow and integrity can be assessed over time in the mammal, or relative to a control not receiving the composition.

It is described that the composition is a pet food, dietary supplement, or a food product formulated for human consumption. The compositions, as well methods for its manufacture and administration as such are identical to that described in the previous aspect of the invention and such disclosure need not be repeated here in its entirety.

It is described that herein the composition administered is a pot food, dietary supplement, or a food product formulated for human consumption, and in numerous embodiments, the mammal is a companion animal. the companion animal is a cat or dog.

The composition administered comprises at least about 1% to about 30% MCTs on a dry weight basis in various applications of the methods. It is described that the administering step is on a regular basis comprising at least once daily. In some presently preferred embodiments the composition is administered as part of a daily dietary regimen for at least about one week. A duration of two, three or even four weeks is also used. Administering the compositions for one to three months, or four months is exemplified herein. It is described that administration will be extended for 4, 5, 6, 7, 8, 9, 10, 11 or even 12 months. In yet longer applications, administration periods extending 1, 2, 3, or more years are anticipated. In such embodiments, it may be useful to at least periodically monitor the animal for ketoacidosis and the like, however, there is no evidence that the compositions or methods provided herein will result in ketoacidosis even after prolonged administration. It is described that the administration of the compositions is maintained for the remainder of the animal's life (for example, the second half of the life expectancy for a animal that has just recently attained aged or geriatric status as defined herein).

It is described that the composition is administered as part of a daily dietary regimen for at least about one week, about three months, or about one year at a minimum.

It is described that the composition administered comprises MCTs in an amount effective for lowering blood urea nitrogen or decreasing protein degradation. In another, the composition comprises MCTs in an amount effective for lowering the amount or activity of alanine aminotransferase.

It is described that the methods described comprise an administration step wherein the composition comprises MCTs in an amount effective for improving social behaviors of a companion animal. Such improvement can comprise interaction with its own or other species, such as a human.

Further described are methods for preventing, reducing, or delaying decline in at least one of cognitive function, motor function, cerebrovascular function, or behavior in a population of healthy aging mammals. Such methods are useful in the development and formulation of diets and nutritional regimens four improving or preventing, reducing or delaying decline in cognitive, motor, or behavioural function. The methods generally comprise:
(a) Identifying a population of healthy aging mammals. In preferred embodiments, the mammals do not have a diagnoses of age-related cognitive impairment, nor obvious indicia of such conditions.
(b) Dividing the population into at least a control group and one or more test groups. The skilled artisan will appreciate that statistical methods of experimental design and available populations of animals may dictate the number of groups into which the sample population can be properly divided.
(c) Formulating at least one diet-based delivery system or regimen for delivering a composition comprising medium chain triglycerides (MCTs) in an amount effective for elevating and maintaining an elevated level of at least one ketone body in the blood of an individual mammal. The formulations of the diet-based delivery system are based on the nutritional/dietary needs of the population including macro and micronutrients, energy requirements, and the like, and further comprise MCTs as part of the diet. Preferably, the MCTs are provided in the food formulation directly, but may also be included as a supplement thereto, in any form previously discussed herein with regard to other aspects of the invention. The MCTs are of the formula [I] as provided above, and as previously wherein the fatty acids esterified to the glycerol backbone are each independently fatty acids having 5-12 carbons.

A particular formulation is described for each individual in the test group on an extended regular basis, as defined herein. Thus, each test group receives a formulation delivering a composition comprising MCTs, while the control group does not receive any composition comprising MCTs but rather receives a comparable formulation lacking MCTs but equivalent in terms of macro and micro nutriments, energy content, fiber, and the like.
(d) Comparing at least one of cognitive function, motor function, cerebrovascular function, or behavior in the control and test groups. Art-known measures and methods can be readily applied here, and the skilled artisan can readily develop additional useful measures of such functions in accordance with the needs of the experiment or population.
(e) Determining which of the diet-based delivery systems for delivering the composition comprising MCTs was effective in preventing, reducing, delaying decline of at least one of cognitive function, motor function, cerebrovascular function, or behavior.
(f) Finally, administering a diet-based delivery system determined in step (e) above to a population of aging mammals, thereby preventing, reducing, delaying decline in at least one of cognitive function, motor function, cerebrovascular function, or behavior.

It is described that the "extended regular basis" for providing the test diet comprises comprises at least once daily for a period (duration) of at least about one week to about one year. Longer durations are contemplated for use herein, and such longer durations amy involve fine-runing prior iterations of formulated delivery systems to improve for example the prevention, reduction, or delay, or to improve palatability, convenience, or the like.

It is described that the methods further comprise the step of monitoring at least one ketone body concentrations in each mammal in the control and test groups. In certain embodiments the amount of each of β-hydroxybutyrate, acetoacetate and acetone is raised.

It is described that the composition delivered by the system or regimen comprises MCTs in an amount effective for lowering the blood level of one or more of alanine, branched chain amino acids, total lipoproteins, unsaturated fatty acids, or VLDL. In It is described that the level of each of alanine, branched chain amino acids, total lipoproteins, unsaturated fatty acids, and VLDL is lowered. It is described that the composition comprises MCTs in an amount effective for raising the blood level of one or more of glutamine, phenylalanine, HDL, or citrate. In yet others, the level of each of glutamine, phenylalanine, HDL, and citrate is raised. In one embodiment wherein the composition comprises MCTs in an amount effective for the lowering the level of each of alanine, branched chain amino acids, total lipoproteins, unsaturated fatty acids, and VLDL, while the level of each of glutamine, phenylalanine, HDL, and citrate is raised.

The described methods, the administered composition comprises MCTs in an amount effective for improving blood flow to the brain, or for improving the integrity of the blood brain barrier, or both. Such improvements in blood flow and integrity can be assessed over time in the mammal, or relative to a control not receiving the composition. They can also be relative, for example, to the control group on average.

It is described that the composition is a pet food, dietary supplement, or a food product formulated for human consumption. The compositions, as well methods for its manufacture and administration as such are identical to that described in the previous description.

It is described that the composition administered is a pet food, dietary supplement, or a food product formulated for human consumption, and in numerous embodiments, the mammal is a companion animal. In certain embodiments, the companion animal is a cat or dog.

The composition administered comprises at least about 1% to about 30% MCTs on a dry weight basis in various applications of the methods, It is described that administering step is on a regular basis comprising at least once daily. In some presently

It is described that the composition is administered as part of a daily dietary regimen for at least about one week. A duration of two, three or even four weeks is also used. Administering the compositions for one to three months, or four months is exemplified herein. It is described that administration will be extended to 4, 5, 6, 7, 8, 9, 10, 11 or even 12 months. In yet longer applications, administration periods extending 1, 2, 3, or more years are anticipated. it may be useful to at least periodically monitor the animal for ketoacidosis and the like, however, there is no evidence that the compositions or methods provided herein will result in ketoacidosis even after prolonged Administration. It is described that the administration of the compositions is maintained for the remainder of the animal's life (for example, the second half of the life expectancy for a animal that has just recently attained aged or geriatric status as defined herein).

It is described that the composition is administered as part of a daily dietary regimen for at least about one week, about three months, or about one year at a minimum.

It is described that the composition administered comprises MCTs in an amount effective for lowering blood urea nitrogen or decreasing protein degradation. In another, the composition comprises MCTs in an amount effective for lowering the amount or activity of alanine aminotransferase.

It is described that the methods provided comprises an administration step wherein the composition comprises MCTs in an amount effective for improving social behaviors of a companion animal. Such improvement can comprise interaction with its own or other species, such as a human.

The following examples are provided to describe the invention in greater detail. The examples are intended illustrate, not to limit, the invention.

### EXAMPLE 1

### Animals and Diets

Fifty-four animals, ranging in age from 8-11 years, were divided into three cognitively-equivalent treatment groups, using errors-to-criterion on tests of object discrimination and reversal learning (Table 1). The animals were free from any pathological condition and were considered healthy aged canines. The first group, the control group, was fed a basal diet consisting of approximately 10% moisture, 26% crude protein, 16% fat, and 6% ash, without any MCT supplementation. The basal diet consisted of ingredients commonly used in companion animal diets, such as brewer rice, chicken, whole wheat, poultry-by-product meal, corn gluten meal, corn grain, animal fat, corn bran, dried egg product, flavor enhancers, vitamins, and minerals. MCTs administered to the animals for these experiments were
greater than 95% of the fatty acids having 8 carbons in the carbon backbone and esterified to the glycerol backbone. The remaining fatty acids were C₆ or C₁₀ containing fatty acids.

The second group received the basal diet supplemented with 1 g/kg/day of the MCTs. The third group received the basal diet supplemented with 2 g/kg/day of MCTs. The test substance was added to the dogs' normal chow and introduced gradually over three days: one-third of the maximum dose ofMCTs was administered on the first day, two-thirds on the second day, and the full dose on the third day. The subjects were maintained on the test substance for the duration of the study. All of the subjects had previously undergone a pre-training protocol to familiarize the animals with the testing apparatus and to provide baseline measures of cognitive ability. For all groups, the amount of food or food-oil mixture fed to the animal was calculated using the formula: kcal rcq = (BW^{0.75}(70)(2), where BW is body weight of the animal in kilograms. This was done to feed the animals the same amount of calories on the basis of BW, and to maintain the BW of each animal across the groups.

The animals began the study in three separate groups, referred to below as cohorts. The first cohort (32 subjects) began the study. The second cohort (10 subjects) started the study about two months later. The third cohort (12 subjects) started the study about three weeks after the second cohort. Cognitive testing began 9 days after the animals began eating the food containing the fell amount of test substance.

Overall, the treatment was generally well tolerated by the animals. Only one animal did not consistently consume the treatment diet consisting of a slurry in which MCTs were combined with the food. As a result, this animal was reassigned to the control group.

Although the planned doses consisted of 1 g/kg/day and 2 g/kg/day, all of the animals may not have received the full dose. Because the MCT oil was manually mixed into the dry kibble, it not only coated the kibble, but also the sides of the bowl. Therefore, animals that did not lick the bowl were not ingesting the full dose. Additionally, animals were required to consume their daily ration within 0.5 hours and not all animals consumed their entire ration on all days.

**Table 1. Subjects and Group Assignments.**

| **Subject** | **ID** | **Sex** | **Cohort** | **Date of Birth** | **Age at Start** | **Errors** | | **Group (g/kg/day)** |
|---|---|---|---|---|---|---|---|---|
| | | | | | | **Discrimination** | **Reversal** | |
| Ani | 2439 | F | 2 | 14-Apr-96 | 8.13 | 39 | 137 | 0 |
| Billy | 38290 | M | 2 | 14-Apr-96 | 8.13 | 54 | 191 | 0 |
| Cindy | 54301 | F | 3 | 20-Feb-96 | 8.32 | 5 | 6 | 0 |
| Clifford | 36948 | M | 1 | 22-Dec-95 | 8.26 | 20 | 95 | 0 |
| Courtney | 37853 | F | 3 | 14-Apr-96 | 8.17 | 37 | 94 | 0 |
| Elmer | 61101 | M | 1 | 12-Apr-95 | 8.95 | 47.5 | 17 | 0 |
| Fudd | 61039 | M | 1 | 20-May-95 | 8.84 | 9 | 75 | 0 |
| Hush | 38275 | M | 1 | 22-Dec-95 | 8.26 | 19 | 11 | 0 |
| Larry | 61078 | M | 1 | 5-Oct-94 | 9.46 | 16 | 82 | 0 |
| Madonna | 54446 | F | 3 | 1-Mar-96 | 8.29 | 23 | 29 | 0 |
| Pebbles | 60669 | F | 1 | 28-Dec-94 | 9.23 | 22 | 80 | 0 |
| P.J. | 2506 | F | 3 | 14-Apr-96 | 8.17 | 85 | 183 | 0 |
| Reggie | 38302 | M | 1 | 22-Dec-95 | 8.26 | 15 | 40 | 0 |
| Scott | 38107 | M | 2 | 14-Apr-95 | 9.11 | 8 | 54 | 0 |
| Sheri | 39033 | F | 3 | 14-Apr-96 | 8.17 | 20 | 113 | 0 |
| Smeagol | 37838 | M | 1 | 22-Dec-95 | 8.26 | 8 | 37 | 0 |
| Tina | 39001 | F | 1 | 22-Dec-95 | 8.26 | 7 | 92 | 0 |
| | | | | **AVG** | **8.49** | **25.56** | **78.59** | |
| | | | | **SEM** | **0.11** | **5.12** | **13.39** | |
| Bear | 61040 | M | 1 | 25-Mar-94 | 9.98 | 20 | 75 | 1 |
| Buddha | 60951 | F | 1 | 12-Mar 95 | 9.03 | 7 | 34 | 1 |
| Curly | 61079 | M | 3 | 2-Jul-94 | 9.93 | 71 | 116 | 1 |
| Eddie | 38448 | M | 2 | 14-Apr-96 | 8.13 | 39 | 64 | 1 |
| Elmo | 38465 | M | 1 | 22-Dec-95 | 8.26 | 18 | 51 | 1 |
| Hitchcock | 38524 | M | 1 | 22-Dec-95 | 8.26 | 18 | 38 | 1 |
| Josephine | 38535 | F | 1 | 22-Dec-95 | 8.26 | 20 | 39.5 | 1 |
| Lionel | 54445 | M | 3 | 1-Mar-96 | 8.29 | 3 | 52 | 1 |
| Liz | 20097 | F | 3 | 14-Apr-95 | 9.16 | 51 | 167 | 1 |
| Louise | 61099 | F | 1 | 25-Jan-95 | 9.16 | 10 | 80 | 1 |
| Marilyn | 38266 | F | 3 | 14-Apr-93 | 11.13 | 46 | 106 | 1 |
| Mia | 38165 | F | 1 | 22-Dec-95 | 8.26 | 29 | 97.5 | 1 |
| Olivia | 38180 | F | 2 | 14-Apr-95 | 9.11 | 32 | 87.5 | 1 |
| Paula | 54362 | F | 3 | 3-Mar-96 | 8.28 | 33 | 21 | 1 |
| Potsie | 39006 | M | 1 | 22-Dec-95 | 8.26 | 13 | 32 | 1 |
| Sarah | 2505 | F | 2 | 14-Apr-94 | 10.10 | 25 | 174.5 | 1 |
| Shadow | 38999 | M | 1 | 22-Dec-95 | 8.26 | 17 | 69 | 1 |
| Speckles | 61098 | F | 1 | 26-Feb-95 | 9.07 | 3 | 50 | 1 |
| | | | | **AVG** | **8.94** | **25.28** | **75.22** | |
| | | | | **SEM** | **0.20** | **4.21** | **10.33** | |
| Boris | 38175 | M | 1 | 22-Dec-95 | 8.26 | 30 | 59 | 2 |
| Chris | 38379 | M | 2 | 14-Apr-95 | 9.11 | 41.5 | 155.5 | 2 |
| Dave | 38037 | M | 2 | 14-Apr-95 | 9.11 | 38 | 95 | 2 |
| Fonzie | 38474 | M | 1 | 22-Dec-95 | 8.26 | 35 | 60 | 2 |
| Genie | 38190 | F | 1 | 22-Dec-94 | 9.25 | 14 | 28 | 2 |
| Janet | 54299 | F | 3 | 20-Feb-95 | 9.31 | 13 | 20 | 2 |
| Jay Lo | 61076 | F | 1 | 1-Jun-95 | 8.81 | 18 | 53 | 2 |
| Kelly | 2446 | F | 2 | 14-Apr-96 | 8.13 | 62 | 185 | 2 |
| Kurt | 39007 | M | 2 | 14-Apr-93 | 11.08 | 37 | 65 | 2 |
| Layla | 39005 | F | 1 | 22-Dec-95 | 8.26 | 3.5 | 155.5 | 2 |
| Linos | 38210 | M | 1 | 22-Dec-95 | 8.26 | 12 | 16 | 2 |
| Lucy | 2636549 | F | 1 | 1-Jul-95 | 8.73 | 14 | 44 | 2 |
| Moe | 61077 | M | 1 | 12-Apr-95 | 8.95 | 35 | 106 | 2 |
| Pippin | 60870 | M | 1 | 6-Aug-95 | 8.64 | 37 | 73 | 2 |
| Sealia | 2056 | F | 1 | 22-Dec-95 | 8.26 | 3 | 47 | 2 |
| Spinner | 38542 | M | 1 | 22-Dec-95 | 8.26 | 17 | 62 . | 2 |
| Suzy Q | 38518 | F | 1 | 22-Dec-95 | 8.26 | 55 | 29 | 2 |
| Thelma | 61075 | F | 1 | 3-Feb-95 | 9.14 | 19.5 | 44 | 2 |
| Tiffany | 54430 | F | 3 | 3-Mar-96 | 8.28 | 22 | 33 | 2 |
| | | | | **AVG** | **8.76** | **26.66** | **70** | |
| | | | | **SEM** | **0.16** | **3.76** | **11.15** | |

### EXAMPLE 2

### Blood Analyses

Blood hematology and biochemistry testing was performed at baseline, day 9 and approximately every 30 days, until the animal completed cognitive testing. Blood chemistry and hematology were monitored as an index of general health, and as a measure of the animals' response to treatment. Additional serum and plasma samples were collected and archived. The animals did not all receive an equal number of blood samples because the animals did not complete the entire study at the same rate. As a result, only the blood hematology and biochemistry obtained at study day 0 (T0), 9 (T1), 39 (T2), 69 (T3), 99 (T4) and 129 (T5) were included in the analysis.

Each blood measure was analyzed using separate repeated-measures ANCOVA with treatment group (0 vs. 1 vs. 2 g/kg/day) as a between-subject variable and time-point (T0 vs. T1 vs.T2 vs. T3 vs. T4 vs. T5) as a within-subject variable. Cohort (1 vs. 2 vs. 3) served as the covariate.

The blood measurements also included screening levels of beta-hydroxybutyrate (BHB). The response to treatment was based on BHB levels, which showed the predicted dose-dependent increase **(****Figure 1****).** As can be seen in Figure 1, the group receiving the larger dose of MCTs (2 g MCT/kg body weight/day) had the highest levels of BHB at all time-points except for baseline (study day 0). The 1 g/kg/day group also had elevated levels of BHB, although they were lower than the 2 g/kg/day group. Similar to humans and rodents fed a ketogenic diet, BHB levels peaked initially and then stabilized at a slightly lower level after approximately 1 month of treatment. Among dogs, individual differences in response to MCT treatments were observed. One animal in each of the treatment groups showed no increase in BHB levels, and were characterized as non-responders. In general, only 5 % of the treated animals did not show a consistent treatment response, as defined by an average treatment BHB level equal or less than the baseline level. This observation has important implications for geriatric dogs, especially given previous reports of dogs being unable to elevate their BHB levels in response to a ketone-generating diet.

Because MCT oil is a high energy nutrient, serum glucose, cholesterol and triglyceride levels were examined. Overall, no treatment-related effects were observed among these variables, although both treatment groups were found to have slightly-elevated cholesterol levels after 3 months (T4) and 4 months (T5) of treatment **(****Figure 5****).**

There was some evidence that the treatment lowered alanine aminotransferase (ALT) levels (see **Figure 2**), although the treatment group by time-point interaction was not statistically significant, and baseline differences in ALT levels could not be ruled out.

Figure 3 reflects that the total protein concentrations tended to be lower in the 1 and 2 g/kg/day groups, compared to the control group (receiving 0 g MCT/kg body weight/day). Total protein was consistently lower in the 2 g/kg/day group, compared to the 0 g/kg/day group. The differences seen were more apparent towards the end of the study **(****Figure 3****).**

Figure 4 shows that blood urea nitrogen (BUN) concentrations can change in response to diet, as can be seen in the data for the group receiving 2 g MCTs/kg/day. Despite being equivalent to the control group at baseline, this high-dose group showed significantly lower amounts of BUN at several time-points during the study **(****Figure 4****).** The 2 g/kg/day group generally had lower BUN concentrations than the control group; they were statistically lower at times T1 (9 days), T2 (39 days), and T4 (99 days). In addition, T4 BUN concentrations in the 2 g/kg/day group were statistically lower than those in control and the 1 g/kg/day groups. BUN concentrations, along with creatinine levels, are commonly used as an index of renal function. Because a change in creatinine in the blood was not observed (data not shown), it is unlikely that renal function was impaired in the treatment animals.

No evidence of a metabolic acidosis was observed in any of the treatment animals. Sodium remained unchanged, and there were no behavioral indices of acidosis (data not shown).

In general, no indications of adverse responses to MCTs were observed in any treatment group, and the animals in all groups remained healthy throughout the study. MCTs may have some beneficial effects on general health, as suggested by the BUN and ALT levels.

### REFERENCE EXAMPLE 3

### Analysis of Treatment Effects on Dog Activity and Behavior

**Activity analyses.** The activity rhythms were measured using the Mini-Mitter® Actiwatch-16® activity monitoring system. Activity counts were recorded every 30 s for a period of 3 days. From these data, average activity levels were calculated for two time periods: (1) sunset to sunrise (night), and (2) sunrise to sunset (day). In addition, the average lag between sunrise and activity onset, defined as a 30-minute bout of activity, was calculated for each animal, with negative scores representing activity onset prior to sunrise.

**Behavioral analysis.** Two separate tests were administered to assess changes in spontaneous, exploratory and social behaviors: the curiosity test and the human interaction test. The tests were administered twice: once prior to treatment onset (baseline) and once after approximately 2 months of treatment. Spontaneous behaviors that were quantified included: total locomotor activity, urination, sniffing, grooming frequency and duration, inactivity, rearing, vocalizing, and jumping. These behaviors were quantified on both the curiosity and human interaction tests. Exploratory behaviors were quantified using the curiosity test, which measured both the duration and the nature of an animal's interaction with objects in its environment. Social behaviors were quantified using the human interaction test.

**Results.** For the curiosity test, animals were placed in the open field arena (approximately 8' x 16') containing 7 objects, and their behaviors were recorded and quantified over a 10-minute period. This test provided measures of spontaneous and exploratory behaviors. On the curiosity test, there was a marginally significant group difference in the amount of locomotor activity [F(2,46) = 2.521, p = 0.091]. The 2 g/kg/day group had greater levels of locomotor activity, when compared to the control and 1 g/kg/day groups, although post-hoc tests did not reach statistical significance (**Figure 6**). A similar trend was observed on the human interaction test although it did not reach statistical significance (**Figure 6**).

**Inactivity.** The curiosity test analysis revealed a significant increase in inactivity on the treatment assessment [F(2,46) = 6.418, p = 0.015]. There also was a significant interaction between treatment group and time [F(2,46) = 3.674, p = 0.033]. Although post-hoc tests did not achieve statistical significance (p > 0.10), the control group showed a large increase in inactivity whereas the 1 and 2 g/kg/day group showed a decrease or very small increase in inactivity between baseline and treatment, respectively (**Figures 8****,** **10**). On the human interaction test, there was significant group effect [F(2,46) = 4.182, p = 0.021] and a trend towards decreased inactivity on the treatment assessment [F(2,46) = 2.760, p = 0.103]. As illustrated in **Figure 9****,** the 1 g/kg/day group was less active than the 2 g/kg/day (p =0.017) and control (p = 0.509) groups.

During the human interaction test, animals were placed in the open field arena (approximately 8' x 16') with a familiar human seated in the center of the room. The human was instructed remain passive and not interact with the dog. The animals' behaviors were recorded and quantified over a 10-minute period. This test provided measures of spontaneous and social behaviors. It was previously reported that the duration and type of interactions with the familiar person is strongly correlated with age and cognitive ability (Siwak *et al.* 2001). Young, cognitively intact dogs typically spend most of the 10-minute period interacting with the human and making physical contact with the human (ex: sitting in the human's lap, nudging the human's arm to get a response). Old, cognitively intact dogs also spend most of their time interacting with the human but without making much physical contact with the person (ex: sitting at the person's feet and looking at them), defined by Siwak *et al.* (2001) as "near time." Demented dogs tend to ignore the person and spend very little time in contact with or near the person.

For each behavior, the data were analyzed using a repeated-measures ANCOVA. Treatment group (0 vs. 1 vs. 2 g/kg/day) was the between-subject variable, study phase (baseline vs. treatment) was the within-subject variable, and cohort (1 vs. 2 vs. 3) was the covariate. *Post-hoc* tests used a Bonferroni correction. A similar analysis that excluded the non-responders also was conducted.

For each behavior, a change from baseline was calculated by subtracting the baseline activity level from the treatment activity level. Positive scores were indicative of an increase in the frequency/duration of a behavior and negative scores were indicative of a decrease. The data from each activity test were analyzed using separate MANCOVAs with group (0 vs. 1 vs. 2 g/kg/day) serving as the between-subject variable and cohort (1 vs. 2 vs. 3) serving as the covariate. The dependent variables were each of the quantified behaviors for a given behavioral test. The analysis was repeated excluding the non-responders.

The behavioral end-points served to determine whether behaviors previously linked to cognition were affected by MCT treatment. A secondary goal was to determine whether other behavioral changes that could be observed.

Treatment with 2 g/kg/day of MCTs had positive effects on spontaneous behaviors. The 2 g/kg/day group had higher total activity levels as assessed using the curiosity test, human interaction test and the Actiwatch devices (**Figures 6****,** **7**). Although the increase in locomotor activity was present at both baseline and during treatment, there also was a treatment-related effect on daytime activity levels using the Actiwatch device. Furthermore, the low dose and control groups showed a decrease in activity with repeated testing whereas the high dose group's activity level remained stable. The decreased activity may partly be due to a habituation to the activity arena. This is unlikely to be the main source of the decline as previous reports have indicated that open field activity remains relatively stable in beagle dogs with repeated testing (Siwak *et al.,* 2001). There also was a significant decrease in rearing in the 2 g/kg/day group (**Figures 11****,** **12**).

**Frequency of Urinating on Objects.** There was an overall group difference in the frequency of urinating on the objects [F(2,46) =4.098, p = 0.023]. As illustrated in **Figure 13****,** the control group urinated on the objects more frequently than either the 1 g/kg/day (p = 0.045) or the 2 g/kg/day (p = 0.049) groups.

**Frequency of Lifting Objects.** There was an overall group difference in the frequency of lifting objects [F(2,46) = 2.571, p =0.087]. The 1 g/kg/day group picked-up objects more frequently when compared to the remaining groups (**Figure 14**), although the post-hoc tests did not reach statistical significance.

Overall, MCT treatment positively modified social behaviors. Both treatment groups showed an increase in the duration of person contact whereas the control group showed a decrease in person contact duration during the treatment assessment. The control group, by contrast, showed an increase in near person duration on the treatment assessment (**Figures 15-18**). Therefore, both treatment groups showed an increase in those social behaviors commonly observed in cognitively intact young dogs. The control group, by contrast, showed a shift in their type of social behavior. The characteristically "young non-demented dog" behaviors were replaced with the "old non-demented dog" behaviors such as spending time near (but not in contact with) the human.

Day and night activity levels in the home cage were observed and recorded using the Actiwatch. Siwak et al. (2003) have shown that aged demented animals show highly irregular activity patterns characterized by increased activity during the night and a larger lag between sunrise and activity onset.

The 2 g/kg/day animals showed a significant increase in daytime activity levels under the treatment condition (**Figure 19**). The remaining groups also showed a small increase in daytime activity levels. This increase was partially attributable to an increase in the number of animals and staff at the facility. The activity onset lag did not yield any treatment effects, however, all of the animals did show a larger lag between sunrise and activity onset during the treatment phase. This effect likely was attributable to seasonal differences. The treatment Actiwatch assessment occurred mainly in the fall, when the sun was rising later in the day. However, staff continued to start their shifts at the same time, which would have been prior to sunrise during the treatment assessment.

### EXAMPLE 4

### Analysis of Treatment Effects on Dog Cognition

The animals were divided into treatment groups based on errors-to-criterion on both discrimination and reversal learning tasks. To ensure that the treatment groups initially were balanced for cognitive ability, three analyses were conducted. For all analyses, task (discrimination vs. reversal) served as the within-subject variable and the dependent variables were errors-to-criterion on the discrimination and the reversal.

**Delayed Non-Match to Position (DNMP).** The first cognitive task, a delayed non-match to position task, provided a measure of both complex learning ability and visuospatial working memory. Briefly, the animal was presented with a block covering a spatial location. The animal had to remember the location over a brief delay period, and select the object covering the novel location after the delay. During the initial training, the delay was fixed at 5s. After learning the task, the animals were tested on a maximal memory protocol in which the delays progressively increased. For the acquisition phase, the raw data consisted of errors, sessions and trials to attain the two-stage learning criterion or, if the animal was unable to learn the task, errors made over 40 sessions (480 trials). For the maximal memory phase, the raw data consisted of the longest delay (in seconds) that the animal was able to pass a two-stage learning criterion within 40 sessions.

The data were analyzed using a MANCOVA with group (0 vs. 1 vs. 2 g/kg) as the between-subject variable and cohort (1 vs. 2 vs. 3) as the covariate. The dependent variables were errors-, sessions-, and trials-to-criterion on the acquisition phase and maximal memory capacity.

The results revealed generally improved learning in the high dose group, as indicated by significant effects on both the sessions- and trials-to-criterion measures. There was no evidence of a dose-dependent effect, as the 1 g/kg/day group was slowest to learn the task (**Figures 20****,** **21**). No significant effects were observed on the maximal memory capacity, although the treatment animals tended to have larger maximal memory capacities (**Figure 22**). These results were partially confounded by the absence of data from four subjects (3 in the 1 g/kg/day group and 1 in the 0 g/kg/day group). The inability of these animals to acquire the task at the 5-second delay resulted in their exclusion from the maximal memory testing, which resulted in the poorest animals being excluded from the two groups.

**Oddity Discrimination.** The second cognitive task assessed complex rule learning and selective attention. The animals had to select the odd object of three (two identical objects and one odd object). The animals received a maximum of 20 sessions to achieve the two-stage learning criterion. In order to better quantify learning, two levels of the oddity were used, with the second being more difficult than the first. Errors-, sessions- and trials-to-criterion or, if an animal was unable to learn within 20 sessions, errors over 20 sessions (400 trials) were used to measure learning at each difficulty level.

The overall treatment effects were not statistically significant. However, on both levels of the oddity discrimination, the animals in the 2 g/kg/day group were learning the task more quickly (**Figures 23****,** **24**). The absence of significance on this task was partially confounded by an inability of many animals to achieve the learning criterion within the allotted time (*i.e.,* a ceiling effect). Furthermore, a chi-square of the pass/fail frequency also supported the better performance by the 2 g/kg/day group.

**Motor Task Acquisition and Performance.** The final cognitive measure consisted of a motor acquisition and performance task. Briefly, the animals were trained to use their paw in order to retrieve a food reward. Initially, the maximal distance at which the animal could successfully retrieve the food was determined. The animals received two sessions at each distance, until they were unable to reach the food reward at least once during a session. Subsequently, performance on the task was assessed by measuring time to reach the food at three distances: the animals' maximal and half maximal distance, and a 0 cm distance that served as control. The raw data on the acquisition phase consisted of the maximal distance, defined as the maximum distance at which the animal could successfully retrieve the food reward, and average latency during the learning sessions (first session at each distance) and average latency during the practice sessions (second session at each distance). The raw data on the performance phase consisted of average latencies to respond at each of the distances (0 cm, half maximum distance, and maximum distance).

The 2 g/kg/day animals were able to achieve longer maximal distances than both the 1 g/kg/day and 0 g/kg/day groups (**Figure 25**). The former comparison was statistically significant. In addition, the 2 g/kg/day seemed to have shorter latencies on their first day of testing at any given distance, although the effect was not significant. Combined, these findings suggest that the animals in the 2 g/kg/day group had improved procedural learning. This interpretation, however, does not take into account any potential effects stemming from dog size. Larger dogs, by nature, have longer paws, and therefore could presumably reach longer distances. It is not likely that this factor significantly impacted the present data because smaller animals were able to reach the same distances as the larger animals.

The expected distance-dependent effect on latencies was observed with the variable motor task. Animals were significantly slower when presented with their half-maximal distance and even slower to respond when presented with their maximal distance. No treatment effects were observed on this phase of the task.

In general, the cognitive end-points suggest that the 2 g/kg/day dose of MCTs has cognitive-enhancing effects but that the 1 g/kg/day was sub-therapeutic. Furthermore, the results on the motor task support the interpretation that the high dose may impact motor ability as a consequence of improved health.

### EXAMPLE 5

### MRI Analysis of Dog Brains and Cerebrovascular System

MRIs were acquired approximately 1.5 months after treatment onset for the first cohort. Images were acquired from 30 test subjects. Since animals from the 2nd and 3rd cohorts had not yet started their treatment period, they were placed in the control group. Table 2 shows the group breakdowns for the MRI analysis. The MRI end-points were a secondary measure aimed at showing that MCTs could induce physiological changes in brain metabolism. Only a subset of the animals in the treatment groups was included because the nature of these measures made it very difficult to obtain a large sample size. For example, if a carotid artery could not be clearly identified in the image, an appropriate control signal could not be used. Nonetheless, the MRI observations provided evidence of physiological changes in the brain after only 1.5 months of MCT treatment.

**Table 2. MRI Subjects and Groups**

| **Animal** | **ID** | **Cohort** | **Group (MRI Analysis only)** |
|---|---|---|---|
| Ani | 2439 | 2 | 0 g/kg/day |
| Billy | 38290 | 2 | 0 g/kg/day |
| Chris | 38379 | 2 | 0 g/kg/day |
| Cindy | 54301 | 3 | 0 g/kg/day |
| Clifford | 36948 | 1 | 0 g/kg/day |
| Courtney | 37853 | 3 | 0 g/kg/day |
| Curly | 61079 | 3 | 0 g/kg/day |
| Dave | 38037 | 2 | 0 g/kg/day |
| Eddie | 38448 | 2 | 0 g/kg/day |
| Elmer | 61101 | 1 | 0 g/kg/day |
| Janet | 54299 | 3 | 0 g/kg/day |
| Kelly | 2446 | 2 | 0 g/kg/day |
| Kurt | 39007 | 2 | 0 g/kg/day |
| Larry | 61078 | 1 | 0 g/kg/day |
| Lionel | 54445 | 3 | 0 g/kg/day |
| Liz | 20097 | 3 | 0 g/kg/day |
| Madonna | 54446 | 3 | 0 g/kg/day |
| Marilyn | 38266 | 3 | 0 g/kg/day |
| Olivia | 38180 | 2 | 0 g/kg/day |
| Paula | 54362 | 3 | 0 g/kg/day |
| Pebbles | 60669 | 1 | 0 g/kg/day |
| P.J. | 2506 | 3 | 0 g/kg/day |
| Reggie | 38302 | 1 | 0 g/kg/day |
| Sarah | 2505 | 2 | 0 g/kg/day |
| Scott | 38107 | 2 | 0 g/kg/day |
| Sheri | 39033 | 3 | 0 g/kg/day |
| Smeagol | 37838 | 1 | 0 g/kg/day |
| Tiffany | 54430 | 3 | 0 g/kg/day |
| Tina | 39001 | 1 | 0 g/kg/day |
| Bear | 61040 | 1 | 1 g/kg/day |
| Buddha | 60951 | 1 | 1 g/kg/day |
| Elmo | 38465 | 1 | 1 g/kg/day |
| Hitchcock | 38524 | 1 | 1 g/kg/day |
| Louise | 61099 | 1 | 1 g/kg/day |

| **Animal** | **ID** | **Cohort** | **Group (MRI Analysis only)** |
|---|---|---|---|
| Mia | 38165 | 1 | 1 g/kg/day |
| Potsie | 39006 | 1 | 1 g/kg/day |
| Shadow | 38999 | 1 | 1 g/kg/day |
| Speckles | 61098 | 1 | 1 g/kg/day |
| Boris | 38175 | 1 | 2 g/kg/day |
| Fonzie | 38474 | 1 | 2 g/kg/day |
| Genie | 38190 | 1 | 2 g/kg/day |
| Jay Lo | 61076 | 1 | 2 g/kg/day |
| Linos | 38210 | 1 | 2 g/kg/day |
| Lucy | 2636549 | 1 | 2 g/kg/day |
| Moe | 61077 | 1 | 2 g/kg/day |
| Pippin | 60870 | 1 | 2 g/kg/day |
| Sealia | 2056 | 1 | 2 g/kg/day |
| Spinner | 38542 | 1 | 2 g/kg/day |
| Suzy Q | 38518 | 1 | 2 g/kg/day |
| Thelma | 61075 | 1 | 2 g/kg/day |

**Blood Volume Index.** The blood volume index (BVI) measured the blood volume in a brain structure using a T1-dynamic MRI and contrast agent (Gd DTPA-BMA, Omniscan®). BVIs were measured for the following four regions: (1) prefrontal-frontal region, (2) thalamus, (3) hippocampus, and (4) cerebellum.

The BVIs were analyzed using a repeated-measures ANOVA with region (prefrontal-frontal cortex vs. thalamus vs. hippocampus vs. cerebellum) serving as the within-subject variable and group (0 g/kg/day vs. 1 g/kg/day vs. 2 g/kg/day) serving as the between-subject variable.

Region-specific differences in blood volume were observed. The prefrontal-frontal cortex had the largest index (most blood volume) whereas the thalamus had the smallest blood volume. The BVI also differed between groups. The 2 g/kg/day group had significantly less blood volume than did the 0 g/kg/day group (**Figure 26**).

**Blood Leakage Index.** The blood leakage index (BLI) measured the leakage of blood out of the brain. The BLI was determined by taking repeated images using a T1-dynamic MRI and contrast agent (Gd DTPA-BMA, Omniscan®) to determine the amount of the contrast agent in the cerebrovascular system as a function of total amount administered. BLIs were measured for the following four regions: (1) prefrontal-frontal region, (2) thalamus, (3) hippocampus, and (4) cerebellum.

The BLI were analyzed using a repeated-measures ANOVA with region (prefrontal-frontal cortex vs. thalamus vs. hippocampus vs. cerebellum) serving as the within-subject variable and group (0 g/kg/day vs. 1 g/kg/day vs. 2 g/kg/day) serving as the between-subject variable.

Similar to the BVI, a region-specific effect was observed, with the prefrontal-frontal cortex having the most leakage and the thalamus having the least leakage. Treatment effects also were apparent on the blood leakage measure. The 2 g/kg/day group had significantly less leakage than the control group (**Figure 27**). These findings suggest that the blood in the high dose animals' brains is not leaking into inappropriate areas of the brain.

**Blood-Brain Barrier Index.** The blood-brain barrier index (BBBI) measured the blood brain barrier leakiness in a brain structure. Higher scores are indicative of more leakage through the blood brain barrier into the brain. BBBI was measured using a T1-dynamic MRI and contrast agent (Gd DTPA-BMA, Omniscan®). BBBIs were measured for the following four regions: (1) prefrontal-frontal region, (2) thalamus, (3) hippocampus, and (4) cerebellum.

The BBBIs were analyzed using a repeated-measures ANOVA with region (prefrontal-frontal cortex vs. thalamus vs. hippocampus vs. cerebellum) serving as the within-subject variable and group (0 g/kg/day vs. 1 g/kg/day vs. 2 g/kg/day) serving as the between-subject variable.

Although no regional differences were observed, there was a marginally significant treatment effect. The 2 g/kg/day group had less BBB leakiness than both the 1 g/kg/day and 0 g/kg/day animals (**Figure 28**).

**Regional Cerebral Blood Volume.** The regional cerebral blood volume index (rCBVI) measured the regional cerebral blood volume in a brain structure using a T2-perfusion MRI and contrast agent (Gd DTPA-BMA, Omniscan®). The rCBVI was measured for both gray and white matter.

The rCBVIs were analyzed using a repeated-measures ANOVA with tissue type (gray vs. white) serving as the within-subject variable and group (0 g/kg/day vs. 1 g/kg/day vs. 2 g/kg/day) serving as the between-subject variable.

A marginally significant treatment effect was observed, with the 2 g/kg/day animals having a larger rCBV than the 0 g/kg/day animals (**Figure 29**).

### EXAMPLE 6

### NMR-Based Nutritional Metabonomics of MCT-Induced Metabolic changes

Samples from the dog studies using control diet, and those with MCT at either 1g or 2g per kg of bodyweight per day were analyzed using NMR to assess metabonomic changes. Metabonomics (also sometimes referred to metabolomics) is the study of quantitative measurement of dynamic multiparametric metabolic response of living systems to changes such as physiological stimuli, or genetic manipulation. The metabolic profile of any given cell, fluid, tissue, organ, or organism at any point in time, or over time can be undertaken with this technology. Powerful methods of statistical analysis are used to help discover differences and treatment effects. The statistical analyses undertaken generally and implemented here include both unsupervised analyses, such as principal component analysis (PCA), and supervised analyses, such as partial least squares discriminant analysis (PLS-DA) and orthogonal partial least squares discriminant analysis (O-PLS-DA). PCA was first used to identify outliers. PLS_DA was used to filter out metabolic information not correlated to the defined classes while allowing useful clustering for metabolic interpretation. The O-PLS-DA was used for further refinement such that the X-matrix (NMR-spectra) and the Y matric (treatment class or time point being tested) are separated into three parts, the first of which is common to X and Y, the second of which contains the specific X variation ("structure noise"), and the third part of which contains the residual variance. O-PLS-DA loading of the data allows better predictive interpretations and more accurate interpretations of the metabolic effects being studied.

Loading plots were constructed to identify the compounds responsible for separation of clusters/groups and the like. Software used included Matlab and Simca P+11.

Plasma samples were measured using conventional 1H-NMR at 660.22 MHz on a Bruker Avance-600 spectrometer. Samples were measured in random order.

The following acquisition parameter were employed:
NOESY-presaturation: D-90°-t₁-90°- acquire free induction decay (FID); where D1 is the relaxation delay (2.0s) during which the water resonance is selectively irradiated, and t₁ is a fixed interval of 3 ms. The water resonance was irradiated for a second time during the mixing time (tm, 100ms).
Carr-Purcell-Meiboom-Gill (CPMG): D₁[-90°-(_{T}-180°- _{T})ₙ-FID]. The spin-echo loop time (2n _{T}) was set at 64 ms. A relaxation time of 2.0s was applied.

For the spectra processing, FIDs were multiplied by an exponential function corresponding to a line broadening (LB) of 0.3 Hz before Fourier transformation for the NOESY presaturation dataset, and 1.0 Hz for the CPMG dataset. Each acquired spectra was checked visually for correct shimming and water suppression followed by correction for phase and baseline distortions using TOPSPIN (version 1.3., Bruker, Karlsruhe, Germany.) Calibration for the chemical shift was preformed using the doublet signal of alpha anomeric proton of glucose at 5.23 ppm.

Peak assignment was done using AMIX viewer (Version 3.6.8, Bruker). The spectra were processed by phasing, baseline and calibration operations prior to being subjected to the statistical analysis described above.

Selected results of the O-PLS-DA analysis are shown in Figures 30, 31, and 32. As can be seen in the Figures, each animal is identified by name, the clusters can be viewed best in color. Figures 30 and 31 each show the same principal component analysis, however Figure 30 has the data for each animal in each group of the 0, 1, and 2 g MCT dose treatment, whereas Figure 31 is simplified by showing only the data for the 0 and the 2 g MCT dose treatments. The separation is easier to see on the less crowded plot. Figure 32 shows the data for the control group (0 g MCT) and the high dose (2 g MCT) for a different set of principal components.

The data allowed the following conclusions to be drawn from the metabolomic study:
Dietary MCT supplementation results in a decrease in the concentrations of alanine, branched-chain amino acids, lipoproteins, unsaturated fatty acids, and urea. MCT supplementation resulted in an expected increase in ketone bodies (β-hydroxy butyrate, acetoacetate, and acetone). It also resulted in an increase in glutamine, and phenylalanine. Also observed were a decrease in lipoproteins, very low density lipoproteins (VLDL) and chylomicrons, and an increase in high density lipoproteins (HDL) and citrate. Further changes were noted over the time course of the study. Increasing with time were the metabolites isobutyric acid, acetic acid, formic acid, and a signal at 3.39 ppm. Lactate, on the other hand, decreased over time.

In conclusion, the metabolomic data both as a function of treatment group and as function of time were valuable for gaining a deeper understanding of metabolic changes with animals receiving dietary MCT for a prolonged time. Together with the behavioral, motor and cognitive function data, and the basic blood chemistry analyses, a detailed picture of the changes that can be attained through such long-term supplementation can be appreciated.

### EXAMPLE 7

### Cats Demonstrate Age-related Congitive Decline

It was also of interest to determine whether a useful model of cognitive function/decline could be developed in other animals. Age-related changes in behavioral signs and brain pathology are reported in cats. There is limited evidence, however, that cats undergo age-related cognitive decline. In both dogs and humans, executive function is impaired early in aging. In the present study, performance of cats of three different age groups on a t-maze test was investigated to examine age-related cognitive changes. The working hypothesis was that performance in reversal learning, a measure of executive function, would decline with increasing age in the cats.

### Materials and Methods:

The subjects were 25 domestic cats divided into three groups based on age. The adult group consisted of 10 subjects between the ages of 3.04 and 4.17 years of age, the old group consisted of 7 cats between the ages of 7.69 and 9.03, and the senior group consisted of 8 cats between the ages of 10.91 and 15.05. The t-maze was a wooden apparatus with four distinct areas; a start box, a run-way and two goal boxes. At the beginning of a trial, the subject was allowed to leave the start area and enter a run-way that branched both to the left and right. A subject made a choice (left or right) when they entered either one of two goal boxes located after the run-way.

In the present study, subjects were tested on three phases. The first phase was a single day in which a subject's preference was determined based on the goal box entered most often. During the second phase, the discrimination phase, subjects were required to run the maze and were only rewarded for choosing their preferred side. Once subjects reached a learning criterion of greater than 80% correct choices, the subjects moved onto the third phase, the reversal phase. In this phase, the rewarded side was switched such that the correct choice was the subject's non-preferred side. Cats were then tested until they reached the learning criteria. Errors on the discrimination and reversal were analyzed using a repeated-measure ANOVA with age group as a between-subject variable and test phase as a within-subject variable.

### Results:

The results are shown in Table 7.1. The results shown are the number of errors committed by the animal at each indicated phase (e.g. discrimination (DISC), reversal (REV), etc) of the study. Significant phase and age effects were found in the initial analysis. As expected, the cats committed more errors on the reversal, than in the discrimination. The age effect observed showed increased overall errors in the aged and senior groups compared to the young group. Additionally, a marginally significant interaction between age-group and test phase was found, as evidenced by increased errors on the reversal by the aged and senior cats compared to the young animals. No differences in discrimination errors were found. Figure 33 shows the analysis of Adult versus Senior cats for the T-Maze task. The "Senior Cats" on the graph are the combined results for the "Old" and "Senior" cats in the Table 7.1.

### Discussion and Conclusion:

The study demonstrated that, like other species, executive function is impaired by age in cats. Additionally, this impairment occurs relatively early in feline aging. The study supports the hypothesis that cats demonstrate age-dependent cognitive decline with aging and that the behavioral changes observed in aged cats may be due to changes in cognitive function and brain aging.

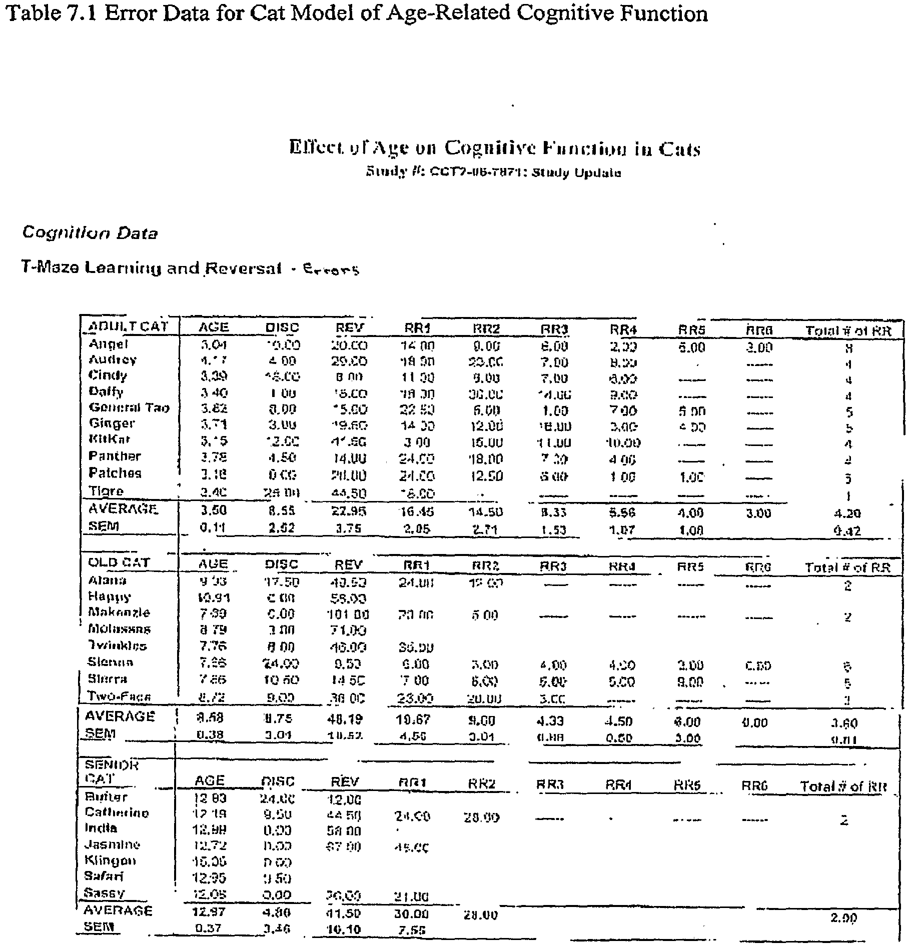

### References:

1. Adams B, Chan ADF, Callahan H, Siwak CT, Tapp D, Ikeda-Douglas C, Atkinson P, Head E, Cotman CW, and Milgram NW (2000a) Spatial learning and memory in the dog as a model of cognitive aging. Behavioural Brain Res. 108:47-56.
2. Adams B, Chan A, Callahan H, and Milgram NW (2000b) The canine as a model of aging and dementia: recent developments. Prog. Neuro-psychopharmacol. Biol. Psychiatry 5:675-692.
3. Chan ADF, Nippak P, Murphey H, Ikeda-Douglas C, Muggenburg B, Head E, Cotman CW, Milgram NW (2002) Visuospatial impairments in aged canines: the role of cognitive-behavioral flexibility. Behavioral Neurosci. 116:443-454.
4. Cotman CW and Berchtold NC (2002) Exercise: a behavioral intervention to enhance brain health and plasticity. Trends Neurosci. 25:295-301.
5. Cummings BJ, Head E, Ruehl W, Milgram NW, and Cotman CW. (1996) The canine as an animal model of human aging and dementia. Neurobiol. Aging 17:259-268.
6. Dimakopoulos AC and Mayer RJ (2002) Aspects of neurodegeneration in the canine brain. J. Nutr. 132:1579S-82S.
7. Drzezga A, Riemenschneider M, Strassner B, Grimmer T, Peller M, Knoll A, Wagenpfeil S, Minoshima S, Schwaiger M, and Kurz A. (2005) Cerebral glucose metabolism in patients with AD and different APOE genotypes. Neurology. 64:102-7.
8. Finch CE, and Cohen DM (1997) Aging, metabolism, and Alzheimer disease: review and hypotheses. Exp. Neurol. 143:82-102.
9. Hoyer S (1990) Brain glucose and energy metabolism during normal aging. Aging (Milano) 2:245-58.
10. London ED, Ohata M, Takei H, French AW, Rapoport SI (1983): Regional cerebral metabolic rate for glucose in beagle dogs of different ages. Neurobiol Aging 4:121-126
11. Munch G, Schinzel R, Loske C, Wong A, Durany N, Li JJ, Vlassara H, Smith MA, Perry G, and Riederer P. (1998) Alzheimer's disease--synergistic effects of glucose deficit, oxidative stress and advanced glycation endproducts. J. Neural. Transm. 105:439-61.
12. Petrells JR, DeCarli C. Dagli M, Grandin CB, Duyn JH, frank JA, Hoffman EA, Theodore WH (1998) Age-related vasodilatory response to acetazolaminde challenge in healthy adults: a dynamic contrast-enhanced MR study. Am. J. Neuroradiol. 19:39-44.
13. Reger MA, Henderson ST, Hale C, Cholerton B, Baker LD, Watson GS, Hyde K, Chapman D, and Craft S. (2004) Effects of beta-hydroxybutyrate on cognition in memory-impaired adults. Neurobiol Aging. 25:311-4.
14. Siwak CT, Tapp PD, and Milgram NW (2001) Effect of age and level of cognitive function on spontaneous and exploratory behaviors in the beagle dog. Learning & Memory. 8:317-25.
15. Siwak CT, Tapp PD, Zicker SC, Murphey HL, Muggenburg BA, Head E, Cotman CW, and Milgram NW (2003) Locomotor activity rhythms in dogs vary with age and cognitive status. Behavioral Neurasci. 117:813-24.
16. Small GW, Ercoli LM, Silverman DH, Huang SC, Komo S, Bookheimer SY, Lavretsky H, Miller K, Siddarth P, Rasgon NL, Mazziotta JC, Saxena S, Wu HM, Mega MS, Cummings JL, Saunders AM, Pericak-Vance MA, Roses AD, Borrio JR, and Phelps ME. (2000) Cerebral metabolic and cognitive decline in persons at genetic risk for Alheimer's disease. Proc. Natl. Acad. Sci. USA. 97:6037-42.
17. Spilt A, Weverling-Rijnsburger A.W, Middelkoop HA, van Der Flier WM, Gissekloo J, de Craen AJ, Bollen EL, Blauw GJ, van Buchom MA, Westendorp RG (2005) Late-onset dementia: structural brain damage and total cerebral blood flow. Radiology 236:990-995.
18. Su M-Y, Head E, Brooks WM, Wang Z, Muggenburg BA, Adam GE, Sutherland RJ, Cotman CW and Nalcioglu O. (1998) MR imaging of anatomic and vascular characteristics in a canine model of human aging. Neurobiol. Aging 19:479-485*.*
19. Swaab DF, Lucassen PJ, Salehi A, Scherder EJ, van Someren EJ, and Verwer RW. - (1998) Reduced neuronal activity and reactivation in Alzheimer's disease. Prog. Brain Res. 117:343-77.
20. Tapp PD, Chu Y, Araujo JA, Chiou JY, Head E, Milgram NW, Su MY (2005) Effects of scopolamine challenge on regional cerebral blood volume. A pharmacological model to validate the use of contrast enhanced magnetic resonance imaging to assess cerebral blood volume in a canine model of aging. Prog. Neuropsychopharmacol. Biol. Psychiatry 29:399-406.
21. Tapp PD, Siwak CT, Estrada J, Muggenburg BA, Head E, Cotman CW, and Milgram NW (2003) Size and reversal learning in the Beagle dog as a measure of executive function and inhibitory control in aging. Learning and Memory 10:64-73.
22. VanItallie TB and Nufert TH (2003) Ketones: metabolism's ugly duckling. Nutr. Rev. 61:327-41.
23. Wardlaw JM, Sandercock PAG, Dennis MS, Starr J (2003) Is breakdown of the blood-brain barrier responsible for Lacunar stroke, leukoaraiosis, and dementia? Stroke 34:806-812.

## Claims

1. A composition comprising medium chain triglycerides (MCTs) composed of fatty acids, each independently having 5-12 carbons, esterified to a glycerol backbone, for use on an extended regular basis in preventing, reducing, or delaying decline in one or more of cognitive function, motor function and cerebrovascular function in an aging mammal that has exceeded 50% of the average lifespan for its particular species,
wherein the composition is used at least once daily for periods in excess of two months and
wherein the mammal is a dog or a cat.

2. The composition for use according to claim 1 wherein greater than 95% of the fatty acids are 8 carbons in length.

3. The composition for use according to claim 2, wherein the remaining fatty acids are 6-carbon or 10-carbon fatty acids.

4. The composition for use according to claim 1, which is a food composition, further comprising on a dry weight basis 15-50% protein, 5-40% fat, 5-1.0°/o ash content, and having a moisture content of 5-20%.

5. The composition for use according to claim 1, comprising at least 1% to 30% MCTs on a dry weight basis.

6. The composition for use according to claim 1, which is a pet food or dietary supplement.

7. Use of a composition comprising medium chain triglycerides (MCTs) composed of fatty acids, each independently having 5-12 carbons, esterified to a glycerol backbone, for the manufacture of a medicament for preventing, reducing, or delaying decline in at least one of cognitive function, motor function and cerebrovascular function in an aging mammal that has exceeded 50% of the average lifespan for its particular species, and wherein the medicament is for administration on an extended regular basis,
wherein administration on an extended regular basis is at least once daily for periods in excess of two months and
wherein the mammal is a dog or a cat.

## Patentansprüche

1. Zusammensetzung, umfassend mittelkettige Triglyceride (MCTs), die aus Fettsäuren zusammengesetzt sind, die jeweils unabhängig voneinander 5-12 Kohlenstoffatome aufweisen, die an einer Glycerolhauptkette verestert sind, zur Verwendung auf langfristiger regelmäßiger Basis zum Verhindern, Reduzieren oder Verzögern des Nachlassens von einem oder mehreren von Denkvermögen, Bewegungsvermögen und zerebrovaskulärer Funktion bei einem alternden Säuger, der 50 % seiner durchschnittlichen Lebensdauer für eine jeweilige Spezies überschritten hat,
wobei die Zusammensetzung mindestens einmal täglich für Zeiträume von mehr als zwei Monaten verwendet wird, und
wobei der Säuger ein Hund oder eine Katze ist.

2. Zusammensetzung zur Verwendung nach Anspruch 1, wobei mehr als 95 % der Fettsäuren eine Länge von 8 Kohlenstoffatomen aufweisen.

3. Zusammensetzung zur Verwendung nach Anspruch 2, wobei die verbleibenden Fettsäuren Fettsäuren mit 6 Kohlenstoffatomen oder Fettsäuren mit 10 Kohlenstoffatomen sind.

4. Zusammensetzung zur Verwendung nach Anspruch 1, die eine Nahrungsmittelzusammensetzung ist, ferner umfassend, basierend auf Trockengewicht, 15-50 % Protein, 5-40 % Fett, 5-10 % Aschegehalt und aufweisend einen Feuchtigkeitsgehalt von 5-20 %.

5. Zusammensetzung zur Verwendung nach Anspruch 1, umfassend mindestens 1 % bis 30 % MCTs, basierend auf Trockengewicht.

6. Zusammensetzung zur Verwendung nach Anspruch 1, die ein Tierfutter oder eine Nahrungsergänzung ist.

7. Verwendung einer Zusammensetzung, umfassend mittelkettige Triglyceride (MCTs), die aus Fettsäuren zusammengesetzt sind, die jeweils unabhängig voneinander 5-12 Kohlenstoffatome aufweisen, die an einer Glycerolhauptkette verestert sind, zur Herstellung eines Medikaments zum Verhindern, Reduzieren oder Verzögern des Nachlassens von mindestens einem von Denkvermögen, Bewegungsvermögen und zerebrovaskulärer Funktion bei einem alternden Säuger, der 50 % seiner durchschnittlichen Lebensdauer für eine jeweilige Spezies überschritten hat, und wobei das Medikament zur Verabreichung auf einer langfristigen regelmäßigen Basis vorgesehen ist,
wobei die Verabreichung auf einer langfristigen regelmäßigen Basis mindestens einmal täglich für Zeiträume von mehr als zwei Monaten erfolgt, und
wobei der Säuger ein Hund oder eine Katze ist.

## Revendications

1. Composition comprenant des triglycérides à chaîne moyenne (TCM) composés d'acides gras, ayant chacun indépendamment 5 à 12 carbones, estérifiés à un squelette glycérol, pour une utilisation sur une base régulière prolongée pour prévenir, réduire ou retarder un déclin d'une ou plusieurs parmi une fonction cognitive, une fonction motrice et une fonction cérébrovasculaire chez un mammifère vieillissant qui a dépassé 50 % de l'espérance de vie moyenne pour son espèce particulière,
où la composition est utilisée au moins une fois par jour pendant des périodes de plus de deux mois et
dans laquelle le mammifère est un chien ou un chat.

2. Composition pour utilisation selon la revendication 1, dans laquelle plus de 95 % des acides gras ont une longueur de 8 carbones.

3. Composition pour utilisation selon la revendication 2, dans laquelle les acides gras restants sont des acides gras à 6 carbones ou à 10 carbones.

4. Composition pour utilisation selon la revendication 1, qui est une composition alimentaire, comprenant en outre sur une base de poids sec 15 à 50 % de protéines, 5 à 40 % de matière grasse, 5 à 10 % de teneur en cendres et ayant une teneur en humidité de 5 à 20 %.

5. Composition pour utilisation selon la revendication 1, comprenant au moins 1 % à 30 % de TCM sur une base de poids sec.

6. Composition pour utilisation selon la revendication 1, qui est un complément alimentaire ou un aliment pour animal de compagnie.

7. Utilisation d'une composition comprenant des triglycérides à chaîne moyenne (TCM) composés d'acides gras, ayant chacun indépendamment 5 à 12 carbones, estérifiés à un squelette glycérol, pour la fabrication d'un médicament pour prévenir, réduire ou retarder le déclin d'au moins l'une parmi une fonction cognitive, une fonction motrice et une fonction cérébrovasculaire chez un mammifère vieillissant qui a dépassé 50 % de l'espérance de vie moyenne pour son espèce particulière et dans laquelle le médicament est destiné à une administration sur une base régulière prolongée,
dans laquelle l'administration sur une base régulière prolongée est d'au moins une fois par jour pendant des périodes de plus de deux mois et
dans laquelle le mammifère est un chien ou un chat.
